# EUROPEAN PATENT SPECIFICATION

(11) **EP 1 824 863 B1**
(45) Date of publication and mention of the grant of the patent: **08.10.2008**
(21) Application number: 05820647.5
(22) Date of filing: 16.12.2005
(51) Int. Cl.: C07F 7/08, A61K 31/695, A61P 31/00, A61P 9/00, A61P 35/00, A61P 5/00

(54) **SILICON COMPOUNDS AND THEIR USE**
SILICIUMVERBINDUNGEN UND DEREN VERWENDUNG
DERIVES DE SILICIUM ET LEUR UTILISATION

(30) Priority: 17.12.2004 GB 0427722
(43) Date of publication of application: 29.08.2007
(73) Proprietor: Takeda Cambridge Limited, Milton Road Cambridge Cambridgeshire CB4 0PA (GB)
(72) Inventor: SHOWELL, Graham Andrew, Takeda Cambridge Limited, Cambridge CB4 0PA (GB); WALSH, Louise Marie, Takeda Cambridge Limited, Cambridge CB4 0PA (GB); MANDAL, Ajay Kumar,Takeda Cambridge Limited, Cambridge CB4 0PA (GB); MILLER, David, John Takeda Cambridge Limited, Cambridge CB4 0PA (GB)
(74) Representative: Perry, Robert Edward
(86) International application number: PCT/GB2005/004905
(87) International publication number: WO 2006/064277

(56) References cited:
- WO-A-20/04045625
- WO-A-20/05005442

## Description

### Field of the Invention

This invention relates to silicon compounds and their use in therapy.

### Background to the Invention

Gonadotropin-Releasing Hormone (GnRH) plays a key role in the biology of reproduction. GnRH is also known as luteinizing hormone-releasing hormone (LH-RH).

The GnRH decapeptide (pyro-Glu-His-Trp-Ser-Tyr-Gly-Leu-Art-Pro-Gly-NH₂ or p-EHWSYGLRPG-NH₂) is formed in neurons of the medical basal hypothalamus from a larger precursor via enzymatic processing. The peptide is released in a pulsatile manner into the pituitary portal circulation system, where GnRH interacts with high-affinity receptors (7-transmembrane G-protein coupled receptors) in the anterior pituitary gland located at the base of the brain. Here, GnRH triggers the release of luteinizing hormone (LH) and follicle-stimulating hormone (FSH), both of which are gonadotropic hormones (gonadotropins). LH stimulates the production of testosterone and estradiol in the testes and ovaries respectively, whilst FSH stimulates follicle growth in women and sperm formation in men. When correctly functioning, the pulsatile release and concentration levels of GnRH are critical for the maintaining of gonadal steroidogenesis and for normal functions of reproduction related to growth and sexual development.

The pituitary response to GnRH varies greatly throughout life. GnRH and the gonadotropins first appear in the foetus at about ten weeks of gestation. Sensitivity to GnRH reduces until the onset of puberty. There is, however, a brief rise during the first three months after birth. Prior to puberty, the FSH response to GnRH is greater than that of LH. Once puberty begins, sensitivity to GnRH increases, and pulsatile LH secretion ensues. Later in puberty and throughout the reproductive years, pulsatile release of GnRH occurs throughout the day, with responsiveness to LH being greater than that of FSH. Pulsatile GnRH release results in pulsatile LH and FSH release and thus testosterone and estradiol release from the gonads. Post-menopause, the concentration of FSH and LH rise, and the post-menopausal levels of FSH are higher than those of LH.

Chronic administration of GnRH agonists and antagonists results in decreased circulating levels of both LH and FSH. GnRH agonists are compounds that mimic endogenous GnRH to stimulate receptors on the pituitary gland, resulting in release of LH and FSH. After a transient rise in gonadal hormone production ("flare" response), the chronic administration of GnRH agonists results in down-regulation of the GnRH receptors. This down-regulation and desensitization results in a reduction in the circulating levels of LH and FSH. In spite of the symptom-exacerbating hormonal flare experienced, GnRH agonists have been the preferred treatment for sex-steroid-dependent pathophysiologies. GnRH agonists have been used to reduce testosterone production, thereby reducing prostate volume in benign prostatic hyperplasia (BPH) and slowing tumour growth in prostate cancer. Such compounds have also been used in the treatment of breast and ovarian cancers.

In recent years, GnRH antagonists have become available for clinical evaluation, and have been shown to have an immediate effect on the pituitary but without the observed flare associated with agonists. Use of GnRH antagonists has been reported for the treatment of ovarian, breast and prostate cancers.

Other uses of antagonists include endometriosis (including endometriosis with pain), uterine myoma, ovarian and mammary cystic diseases (including polycystic ovarian disease), prostatic hypertrophy, amenorrhoea (e.g. secondary amenorrhoea), and precocious puberty. These compounds may also be useful in the symptomatic relief of premenstrual syndrome (PMS). Antagonists may also be useful to regulate the secretion of gonadotropins in male mammals to arrest spermatogenesis (e.g. as male contraceptives), and for treatment of male sex offenders. GnRH antagonists and agonists have been shown to have utility in treatments where a reversible suppression of the pituitary-gonadal axis is desired.

The presence of GnRH receptors on anterior pituitary cells and several tumour cell types offers the opportunity to develop drugs that act upon receptors to treat both hormone-dependent and hormone-independent cancers.

Conventionally, androgen deprivation has been the most effective systematic therapy for the treatment of metastatic carcinoma of the prostate. The prostate gland requires androgens for normal growth, maintenance, and function. Prostate cancer and benign prostate hyperplasia, however, are common in men and develop in an environment of continuous exposure to androgen. Utilizing a GnRH antagonist to interrupt the pituitary-gonadal axis reduces androgen production and results in tumour growth modulation.

GnRH antagonists may have a direct effect on tumour growth by blocking receptors on the tumour cells. For those cancer types that respond both to sex hormones and to GnRH directly, antagonists should be effective in slowing tumour growth by two mechanisms. Since GnRH receptors are present on many prostate and breast cancer cells, it has recently been proposed that GnRH antagonists may also be effective in treating non-hormone-dependent tumours. Recent literature examples indicate that GnRH receptors are present on a number of cancer cell lines. In particular, prostate, ovarian and breast cancers (see for example Montagnani et al., Arch. Ital, Urol. Androl. 1997, 69(4), 257-263; Jungwirth et al., Prostate 1997, 32(3), 164-172; Srkalovic et al., Int. J. Oncol. 1998, 12(3), 489-498; Kottler et al., Int. J. Cancer 1997, 71(4), 595-599.

Available GnRH antagonists have primarily been peptide analogues of GnRH (see, for example, WO93/03058). Peptide antagonists of peptide hormones have some potency but, the use of current peptide antagonists is often associated with problems because peptides are degraded by physiological enzymes and often poorly distributed within the organism being treated. They thus have a limited effectiveness as drugs.

WO00/20358 discloses non-peptide analogues of GnRH.

Sila-substitution (C/Si-exchange) of drugs is a relatively recent approach for searching for organo-silicon compounds, which have beneficial biological properties. The approach involves the replacement of specific carbon atoms in compounds by silicon, and monitoring how the biological properties of the compounds have changed. A review of this approach is provided in Tacke and Zilch, Endeavour, New Series, 10, 191-197 (1986). WO2004/045625 discloses silicon-containing compounds that are used in the manufacture of a medicament in relation to a disease or condition associated with GnRH.

### Summary of the Invention

A first aspect of the invention is a compound of formula (I), (II), (III), (IV) or (V): 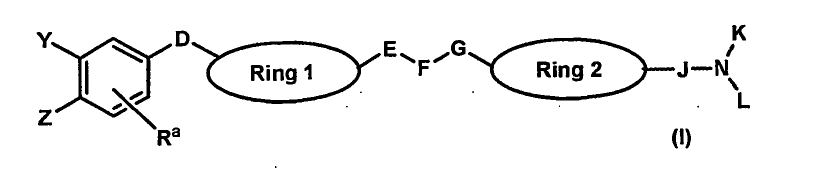 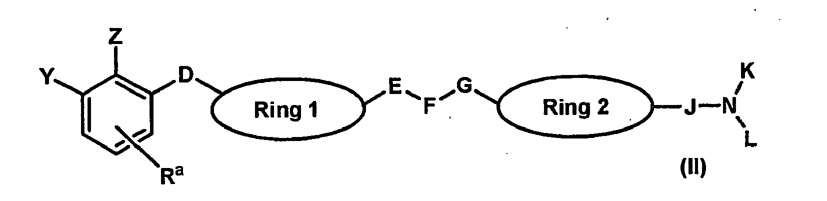 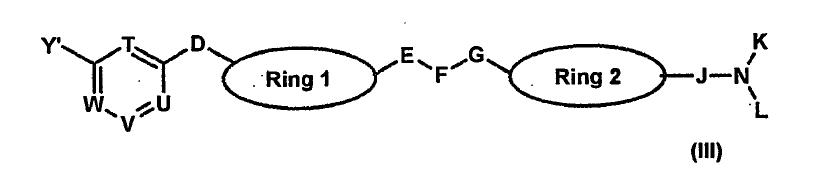 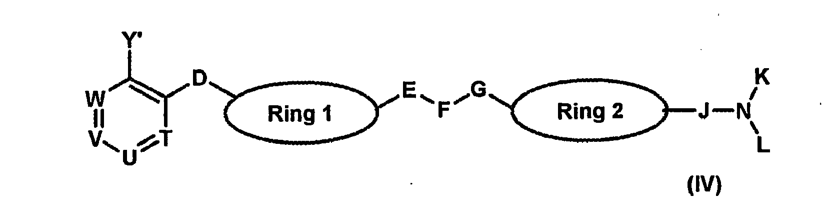 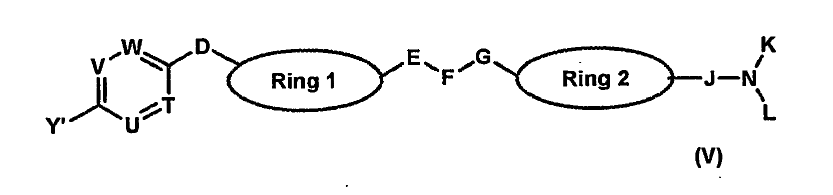 wherein
D is -(CH₂)ₙ-, -C(=X)-, -O-, -S(O)ₘ-, -C(=X)N(R^{e})-, -C(R^{b})₂-, or -C(R^{b})=C(R^{b})-, -CH(R^{b})CH(R^{b})-;
E is optionally present and is -(CH₂)ₙ-, -N(R^{d})-, -(CH₂)ₙN(R^{d})- or -N(R^{d})(CH₂)ₙ-;
F is -C(=X)- or -N(R^{d})-;
G is -(CH₂)ₙ-, -N(R^{d})-, -(CH₂)ₙN(R^{d})- or -N(R^{d})(CH₂)ₙ;
J is a bond, a bridged heterocycloalkyl or a group of formula (i) or formula (ii): 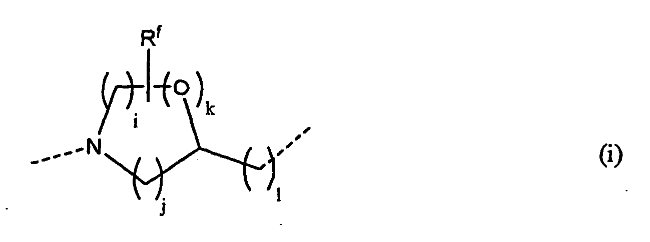 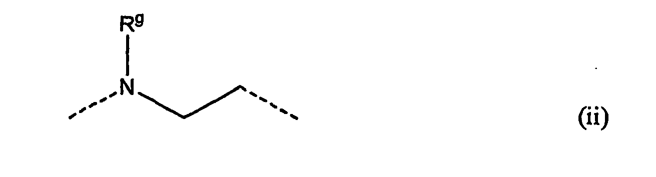 wherein
R^{f} is optionally present and is oxo (=O);
R^{g} is alkyl or -C(O)-alkyl;
i and j are the same or different and are each 1 or 2; and
k and I are the same or different and are each 0 or 1;
K and L are the same or different and are each hydrogen, alkyl, cycloalkyl, -alkyl-cycloalkyl or heterocycloalkyl optionally substituted with R^{a};
or K and L taken together form heterocycloalkyl;
each R^{a} is the same or different and is hydrogen, halogen, alkyl, aryl, hydroxy, alkoxy, -alkoxy-(CH₂)ₙC(=O)OR^{b}, -O-aryl, -C(=X)R^{c}, -NO₂, -CN, -N(R^{c})C(=X)R^{c}, -C(=X)N(R^{c})₂, -S(O)₂N(R^{c})₂ or -N(R^{e})₂;
each R^{b} is the same or different and is hydrogen or alkyl;
each R^{c} is the same or different and is alkyl, cycloalkyl, -alkyl-aryl, -alkyl-cycloalkyl or aryl optionally substituted with R^{a};
each R^{d} is the same or different and is hydrogen, alkyl or aryl optionally substituted with R^{a};
each R^{e} is the same or different and is hydrogen, alkyl; or R^{e} is aryl or heteroaryl, either of which is optionally substituted with R^{a};
one or two of T, U, V and W is nitrogen, and the others are each C(R^{a});
each X is the same or different and is oxygen or sulphur;
Y and Z are the same or different and are each hydrogen, halogen, alkyl, hydroxy, alkoxy, -CN, -N(R^{d})C(=X)R^{c}, -C(=X)N(R^{c})(R^{d}), -S(O)ₘ-R^{c}, -N(R^{c})(R^{d})S(O)₂, -S(O)₂N(R^{c})(R^{d}), -N(R^{e})₂, -Si(R^{c})₃, -alkyl-Si(R^{c})₃, aryl optionally substituted with R^{a} or -O-aryl optionally substituted with R^{a};
Y' is -Si(R^{c})₃ or -alkyl-Si(R^{c})₃;
Rings 1 and 2 are the same or different and are each arylene or heteroarylene, either of which is optionally substituted with R^{a};
each m is the same or different and is 0, 1 or 2; and
each n is the same or different and is 0, 1, 2 or 3;
with the provisos that: at least one of Y and Z comprises a silicon atom; the compound does not contain a N-N single bond; and the compound is not 5-[2-bromo-5-(trimethylsilyl)phenoxy]-*N*-{2-[(2-aminoethyl)propylamino)]-4,6-dimethoxy-1,3-pyrimidin-5-yl}furan-2-carboxamide as disclosed in WO2005/005442;
or a pharmaceutically acceptable salt thereof.

Compounds of the invention may act as GnRH antagonists and, as a result, may have utility in cancer therapy or in the treatment or prevention of endometriosis, uterine myoma, an ovarian disease, a mammary cystic disease, prostatic hypertrophy, amenorrhoea, precocious puberty, premenstrual syndrome, a sex-steroid-dependent pathophysiology, benign prostatic hyperplasia or Alzheimer's disease, or to arrest spermatogenesis.

Accordingly, a second aspect of the invention is the use of a compound of the invention for the manufacture of a medicament for cancer therapy or for the treatment or prevention of endometriosis, uterine myoma, an ovarian disease, a mammary cystic disease, prostatic hypertrophy, amenorrhoea, precocious puberty, premenstrual syndrome, a sex-steroid-dependent pathophysiology, benign prostatic hyperplasia or Alzheimer's disease, or to arrest spermatogenesis.

Another aspect of the invention is a pharmaceutical composition comprising a compound of the invention and a pharmaceutically acceptable diluent or carrier.

### Description of the Invention

Certain compounds and combinations of substituents are preferred; in particular see the subclaims.

The term "alkyl" as used herein refers to an optionally substituted straight or branched chain alkyl moiety having from one to six carbon atoms. The term includes, for example, methyl, ethyl, propyl, isopropyl, butyl, tert-butyl, pentyl, hexyl and the like. The substituents may be the same or different in each occurrence and selected from halogen and the like. "C₁₋₆ alkyl" has the same meaning. "Alkylene" refers to a similar, divalent group.

The term "alkoxy" as used herein refers to an optionally substituted straight or branched chain alkoxy group containing one to six carbon atoms. The term includes, for example, methoxy, ethoxy, propoxy, isopropoxy, butoxy, tert-butoxy, pentoxy, hexoxy and the like. The substituents may be the same or different in each occurrence and selected from halogen and the like. "C₁₋₆ alkoxy" has the same meaning.

The term "halogen" as used herein refers to F, Cl, Br or I.

The term "aryl" as used herein refers to optionally substituted aromatic ring systems comprising six to ten ring atoms, and optionally substituted polycyclic ring systems having two or more cyclic rings at least one of which is aromatic. This term includes, for example, phenyl and naphthyl. The group may be optionally substituted with the substituents being the same or different in each occurrence and selected from R^{a} and the like. "Arylene" refers to a similar, divalent group.

The term "cycloalkyl" as used herein refers to a saturated alicyclic moiety having from three to six carbon atoms. The term includes, for example, cyclopropyl, cyclobutyl, cyclopentyl, cyclohexyl and the like. The group may be optionally substituted by any substituent described herein. "Cycloalkylene" refers to a similar, divalent group.

The term "cycloalkenyl" as used herein refers to an alicyclic moiety having from three to six carbon atoms and having in addition at least one double bond. The term includes, for example, cyclopentenyl, cyclohexenyl and the like. The group may be optionally substituted by any substituent described herein. "Cycloalkenylene" refers to a similar, divalent group.

The term "heterocycloalkyl" as used herein refers to a saturated heterocyclic moiety having from three to seven carbon atoms and one or more heteroatoms selected from the group N, O, S, P and Si. The term includes, for example, azetidinyl, pyrrolidinyl, tetrahydrofuranyl, piperidinyl and the like. The group may be optionally substituted by any substituent described herein. "Heterocycloalkylene" refers to a similar, divalent group.

The term "heteroaryl" as used herein refers to aromatic ring systems of five to ten atoms at least one atom of which is selected from O, N and S. The term includes, for example, furanyl, thiophenyl, pyridyl, indolyl, quinolyl and the like. The group may be optionally substituted with R^{a} and the like. "Heteroarylene" refers to a similar, divalent group.

The term "bridged heterocycloalkyl" as used herein refers to a fused bicyclic heterocycloalkyl moiety having from 6 to 12 carbon atoms and one or two heteroatoms selected from N, O, S and Si. The term includes, for example, tropanyl and isoquinuclidinyl. The conformation of the group may range from [2.1.1] to [4.3.3] system. The group may be optionally substituted with one or more substituents, the substituents being the same or different in each occurrence and selected from alkyl, alkoxy and the like.

Preferred compounds of the invention include:
N-(2-(4-aminopiperidin-1-yl)-4,6-dimethoxypyrimidin-5-yl)-5-(2-methyl-5-(trimethylsilyl)phenoxy)furan-2-carboxamide;
N-(4,6-dimethoxy-2-(4-(methylamino)piperidin-1-yl)pyrimidin-5-yl)-5-(2-methyl-5-(trimethylsilyl)phenoxy)furan-2-carboxamide;
N-(2-(4-(dimethylamino)piperidin-1-yl)-4,6-dimethoxypyrimidin-5-yl)-5-(2-methyl-5-(trimethylsilyl)phenoxy)furan-2-carboxamide;
N-(2-(4-(azetidin-1-yl)piperidin-1-yl)-4,6-dimethoxypyrimidin-5-yl)-5-(2-methyl-5-(trimethylsilyl)phenoxy)furan-2-carboxamide;
N-(2-(4-(dimethylamino)piperidin-1-yl)-4,6-dimethoxypyrimidin-5-yl)-5-(3-(trimethylsilyl)phenoxy)furan-2-carboxamide;
N-(2-(4-(dimethylamino)piperidin-1-yl)-4,6-dimethoxypyrimidin-5-yl)-5-(3-(ethyldimethylsilyl)phenoxy)furan-2-carboxamide;
N-(2-(3-aminoazetidin-1-yl)-4,6-dimethoxypyrimidin-5-yl)-5-(2-methyl-5-(trimethylsilyl)phenoxy)furan-2-carboxamide;
N-(4,6-dimethoxy-2-(3-(methylamino)azetidin-1-yl)pyrimidin-5-yl)-5-(2-methyl-5-(trimethylsilyl)phenoxy)furan-2-carboxamide;
N-(2-(3-(dimethylamino)azetidin-1-yl)-4,6-dimethoxypyrimidin-5-yl)-5-(2-methyl-5-(trimethylsilyl)phenoxy)furan-2-carboxamide;
N-(2-(3-(azetidin-1-yl)azetidin-1-yl)-4,6-dimethoxypyrimidin-5-yl)-5-(2-methyl-5-(trimethylsilyl)phenoxy)furan-2-carboxamide;
N-(2-(3-(azetidin-1-yl)azetidin-1-yl)4,6-dimethoxypyrimidin-5-yl)-5-(3-(trimethylsilyl)phenoxy)furan-2-carboxamide;
N-(2-(3-(azetidin-1-yl)azetidin-1-yl)-4,6-dimethoxypyrimidin-5-yl)-5-(3-(ethyldimethylsilyl)phenoxy)furan-2-carboxamide;
N-(2-(3-aminopyrrolidin-1-yl)-4,6-dimethoxypyrimidin-5-yl)-5-(2-methyl-5-(trimethylsilyl)phenoxy)furan-2-carboxamide;
N-(4,6-dimethoxy-2-(3-(methylamino)pyrrolidin-1-yl)pyrimidin-5-yl)-5-(2-methyl-5-(trimethylsilyl)phenoxy)furan-2-carboxamide;
N-(2-(3-(dimethylamino)pyrrolidin-1-yl)-4,6-dimethoxypyrimidin-5-yl)-5-(2-methyl-5-(trimethylsilyl)phenoxy)furan-2-carboxamide;
N-(2-(3-(azetidin-1-yl)pyrrolidin-1-yl)-4,6-dimethoxypyrimidin-5-yl)-5-(2-methyl-5-(trimethylsilyl)phenoxy)furan-2-carboxamide;
N-(2-(3-(dimethylamino)pyrrolidin-1-yl)-4,6-dimethoxypyrimidin-5-yl)-5-(3-(trimethylsilyl)phenoxy)furan-2-carboxamide;
N-(2-(3-(dimethylamino)pyrrolidiri-1-yl)-4,6-dimethoxypyrimidin-5-yl)-5-(3-.(ethyldimethylsilyl)phenoxy)furan-2-carboxamide;
N-(2-(3-(aminomethyl)pyrrolidin-1-yl)-4,6-dimethoxypyrimidin-5-yl)-5-(2-methyl-5-(trimethylsilyl)phenoxy)furan-2-carboxamide;
N-(4,6-dimethoxy-2-(3-((methylamino)methyl)pyrrolidin-1-yl)pyrimidin-5-yl)-5-(2-methyl-5-(trimethylsilyl)phenoxy)furan-2-carboxamide;
N-(2-(3-((dimethylamino)methyl)pyrrolidin-1-yl)-4,6-dimethoxypyrimidin-5-yl)-5-(2-methyl-5-(trimethylsilyl)phenoxy)furan-2-carboxamide;
N-(2-(3-(azetidin-1-ylmethyl)pyrrolidin-1-yl)-4,6-dimethoxypyrimidin-5-yl)-5-(2-methyl-5-(trimethylsilyl)phenoxy)furan-2-carboxamide;
N-(2-(3-((dimethylamino)methyl)pyrrolidin-1-yl)-4,6-dimethoxypyrimidin-5-yl)-5-(3-(trimethylsilyl)phenoxy)furan-2-carboxamide;
N-(2-(3-((dimethylamino)methyl)pyrrolidin-1-yl)-4,6-dimethoxypyrimidin-5-yl)-5-(3-(ethyldimethylsilyl)phenoxy)furan-2-carboxamide;
N-(2-(2-(aminomethyl)morpholin-4-yl)-4,6-dimethoxypyrimidin-5-yl)-5-(2-methyl-5-(trimethylsilyl)phenoxy)furan-2-carboxamide;
N-(4,6-dimethoxy-2-(2-((methylamino)methyl)morpholin-4-yl)pyrimidin-5-yl)-5-(2-methyl-5-(trimethylsilyl)phenoxy)furan-2-carboxamide;
N-(2-(2-((dimethylamino)methyl)morpholin-4-yl)-4,6-dimethoxypyrimidin-5-yl)-5-(2-methyl-5-(trimethylsilyl)phenoxy)furan-2-carboxamide;
N-(2-(2-(azetidin-1-ylmethyl)morpholin-4-yl)-4,6-dimethoxypyrimidin-5-yl)-5-(2-methyl-5-(trimethylsilyl)phenoxy)furan-2-carboxamide;
N-(2-(2-((dimethylamino)methyl)morpholin-4-yl)-4,6-dimethoxypyrimidin-5-yl)-5-(3-(trimethylsilyl)phenoxy)furan-2-carboxamide;
N-(2-(2-((dimethylamino)methyl)morpholin-4-yl)-4,6-dimethoxypyrimidin-5-yl)-5-(3-(ethyldimethylsilyl)phenoxy)furan-2-carboxamide;
N-(2-(4-(dimethylamino)-2-oxopiperidin-1-yl)-4,6-dimethoxypyrimidin-5-yl)-5-(2-methyl-5-(trimethylsilyl)phenoxy)furan-2-carboxamide;
N-(4,6-dimethoxy-2-(4-(methylamino)-2-oxopiperidin-1-yl)pyrimidin-5-yl)-5-(2-methyl-5-(trimethylsilyl)phenoxy)furan-2-carboxamide;
N-(2-(4-(dimethylamino)-2-oxopiperidin-1-yl)-4,6-dimethoxypyrimidin-5-yl)-5-(3-(trimethylsilyl)phenoxy)furan-2-carboxamide;
N-(4,6-dimethoxy-2-(4-(methylamino)-2-oxopiperidin-1-yl)pyrimidin-5-yl)-5-(3-(trimethylsilyl)phenoxy)furan-2-carboxamide;
N-(2-(4-(dimethylamino)-2-oxopiperidin-1-yl)-4,6-dimethoxypyrimidin-5-yl)-5-(3-(ethyldimethylsilyl)phenoxy)furan-2-carboxamide;
N-(2-((2-aminoethyl)(propyl)amino)-4,6-dimethoxypyrimidin-5-yl)-5-(2-methyl-5-(trimethylsilyl)phenoxy)furan-2-carboxamide;
N-(4,6-dimethoxy-2-((2-(methylamino)ethyl)(propyl)amino)pyrimidin-5-yl)-5-(2-methyl-5-(trimethylsilyl)phenoxy)furan-2-carboxamide;
N-(2-((2-(dimethylamino)ethyl)(propyl)amino)-4,6-dimethoxypyrimidin-5-yl)-5-(2-methyl-5-(trimethylsilyl)phenoxy)furan-2-carboxamide;
N-(2-((2-(azetidin-1-yl)ethyl)(propyl)amino)-4,6-dimethoxypyrimidin-5-yl)-5-(2-methyl-5-(trimethylsilyl)phenoxy)furan-2-carboxamide;
N-(2-((2-(dimethylamino)ethyl)(propyl)amino)-4,6-dimethoxypyrimidin-5-yl)-5-(3-(trimethylsilyl)phenoxy)furan-2-carboxamide;
N-(2-((2-(dimethylamino)ethyl)(propyl)amino)-4,6-dimethoxypyrimidin-5-yl)-5-(3-(ethyldimethylsilyl)phenoxy)furan-2-carboxamide;
N-(2-((2-aminoethyl)(ethyl)amino)-4,6-dimethoxypyrimidin-5-yl)-5-(2-methyl-5-(trimethylsilyl)phenoxy)furan-2-carboxamide;
N-(2-(ethyl(2-(methylamino)ethyl)amino)-4,6-dimethoxypyrimidin-5-yl)-5-(2-methyl-5-(trimethylsilyl)phenoxy)furan-2-carboxamide;
N-(2-((2-(dimethylamino)ethyl)(ethyl)amino)-4,6-dimethoxypyrimidin-5-yl)-5-(2-methyl-5-(trimethylsilyl)phenoxy)furan-2-carboxamide;
N-(2-((2-(azetidin-1-yl)ethyl)(ethyl)amino)-4,6-dimethoxypyrimidin-5-yl)-5-(2-methyl-5-(trimethylsilyl)phenoxy)furan-2-carboxamide;
N-(2-((2-(dimethylamino)ethyl)(ethyl)amino)-4,6-dimethoxypyrimidin-5-yl)-5-(3-(trimethylsilyl)phenoxy)furan-2-carboxamide;
N-(2-((2-(dimethylamino)ethyl)(ethyl)amino)-4,6-dimethoxypyrimidin-5-yl)-5-(3-(ethyldimethylsilyl)phenoxy)furan-2-carboxamide;
N-(2-(N-(2-(dimethylamino)ethyl)propionamido)-4,6-dimethoxypyrimidin-5-yl)-5-(2-methyl-5-(trimethylsilyl)phenoxy)furan-2-carboxamide;
N-(2-(N-(2-(dimethylamino)ethyl)acetamido)-4,6-dimethoxypyrimidin-5-yl)-5-(2-methyl-5-(trimethylsilyl)phenoxy)furan-2-carboxamide;
N-(2-(N-(2-(dimethylamino)ethyl)propionamido)-4,6-dimethoxypyrimidin-5-yl)-5-(3-(trimethylsilyl)phenoxy)furan-2-carboxamide;
N-(2-(N-(2-(dimethylamino)ethyl)acetamido)-4,6-dimethoxypyrimidin-5-yl)-5-(3-(trimethylsilyl)phenoxy)furan-2-carboxamide;
N-(2-(N-(2-aminoethyl)propionamido)-4,6-dimethoxypyrimidin-5-yl)-5-(2-methyl-5-(trimethylsilyl)phenoxy)furan-2-carboxamide;
N-(2-(N-(2-aminoethyl)acetamido)-4,6-dimethoxypyrimidin-5-yl)-5-(2-methyl-5-(trimethylsilyl)phenoxy)furan-2-carboxamide;
5-(2-methyl-5-(trimethylsilyl)phenoxy)-N-(2-(3-(dimethylamino)-8-aza-bicyclo[3.2.1]octan-8-yl)-4,6-dimethoxypyrimidin-5-yl)furan-2-carboxamide;
N-(2-(ethyl(2-(isopropylamino)ethyl)amino)-4,6-dimethoxypyrimidin-5-yl)-5-(2-methyl-5-(trimethylsilyl)phenoxy)furan-2-carboxamide;
N-(2-(3-(isopropylamino)pyrrolidin-1-yl)-4,6-dimethoxypyrimidin-5-yl)-5-(2-methyl-5-(trimethylsilyl)phenoxy)furan-2-carboxamide;
N-(4,6-Dimethoxy-2-(methyl(1-methylpyrrolidin-3-yl)amino)pyrimidin-5-yl)-5-(2-methyl-5-(trimethylsilyl)phenoxy)furan-2-carboxamide; and
N-(4,6-dimethoxy-2-(imidazolidin-2-on-1-yl)pyrimidin-5-yl)-5-(2-methyl-5-(trimethylsilyl)phenoxy)furan-2-carboxamide;
the corresponding structures of which are shown below, respectively:

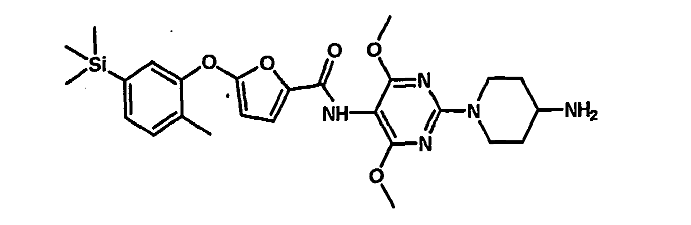

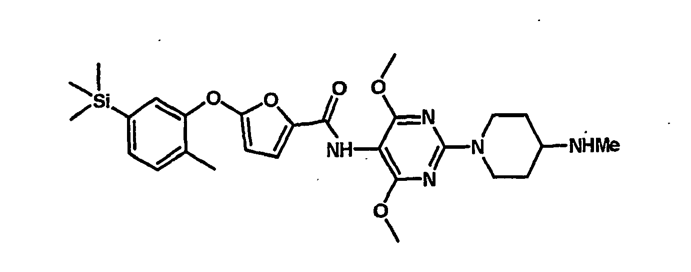

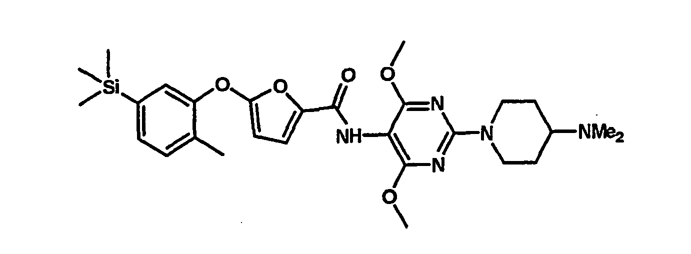

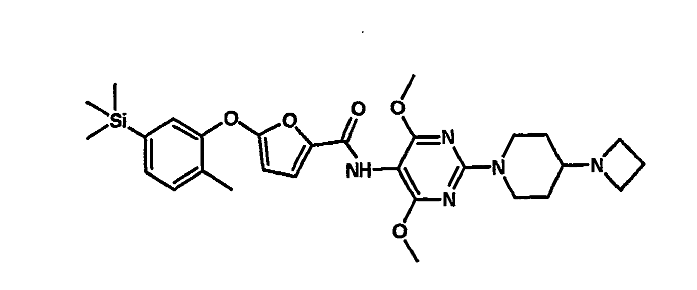

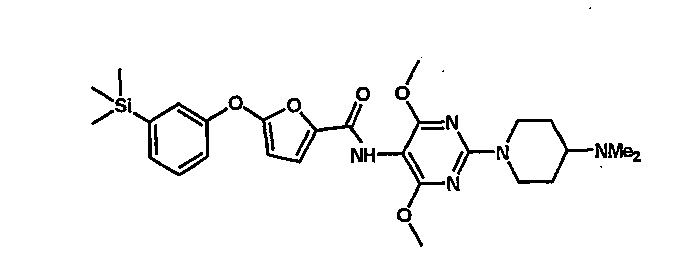

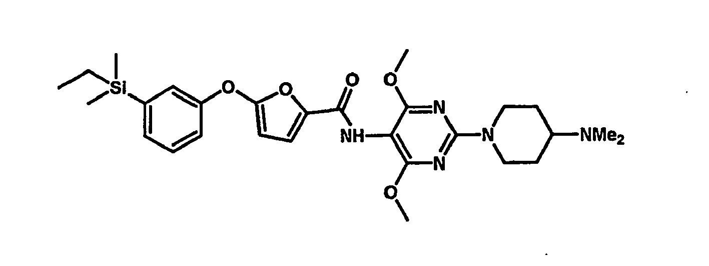

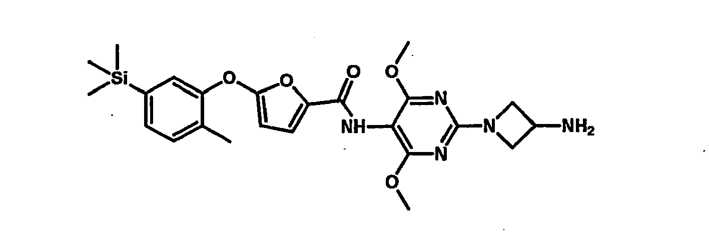

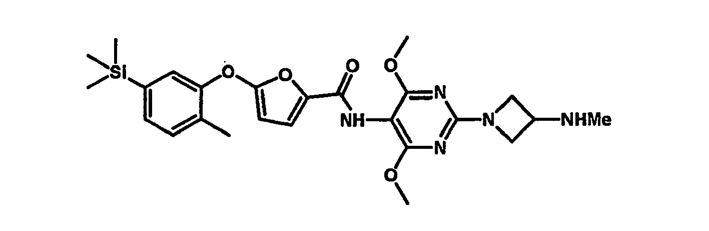

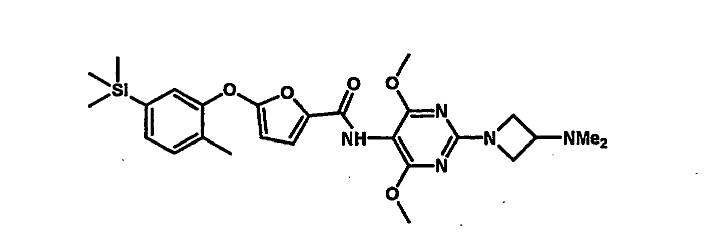

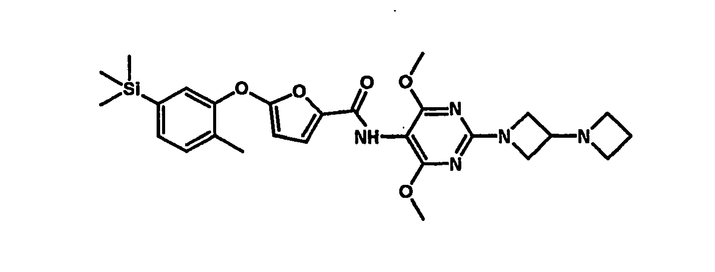

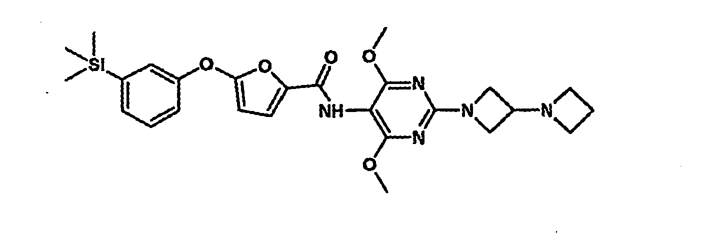

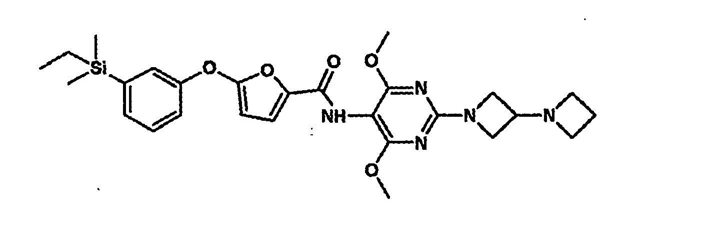

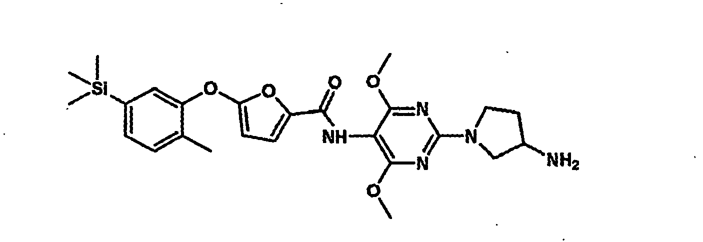

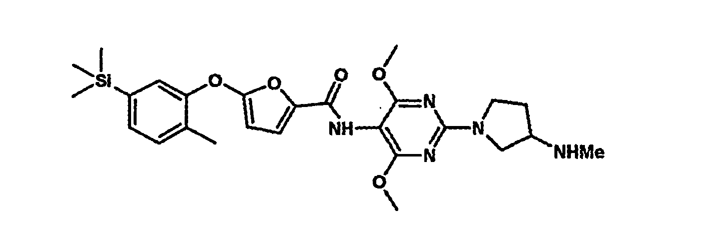

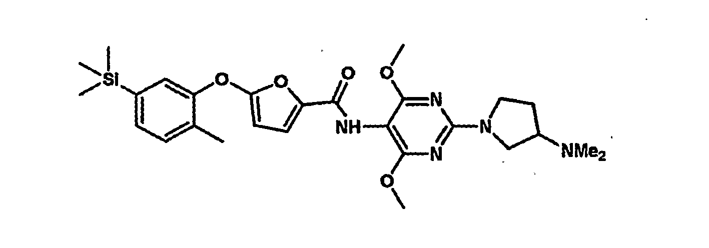

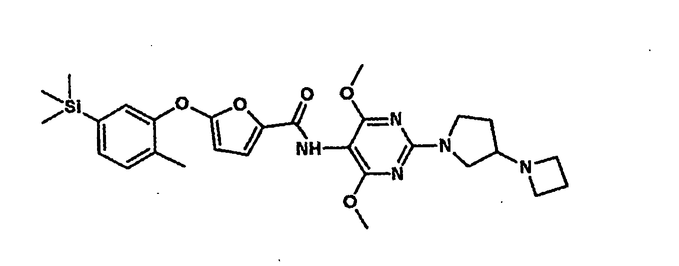

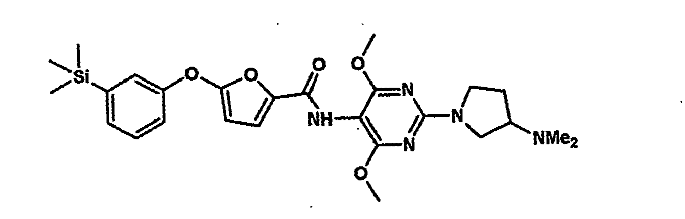

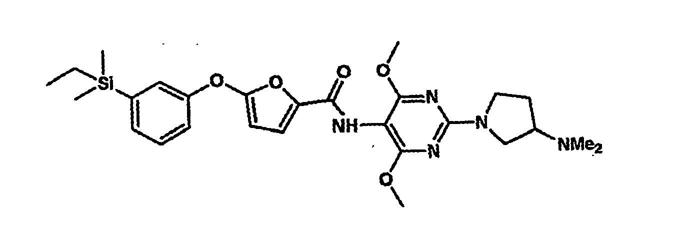

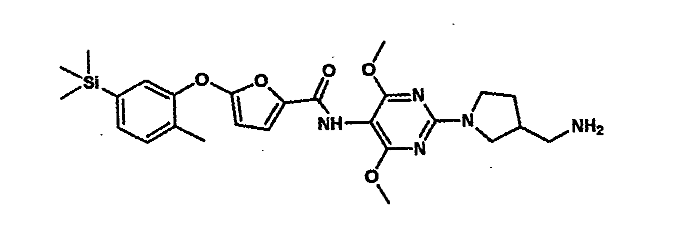

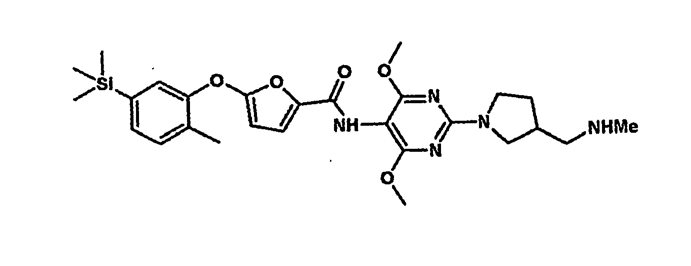

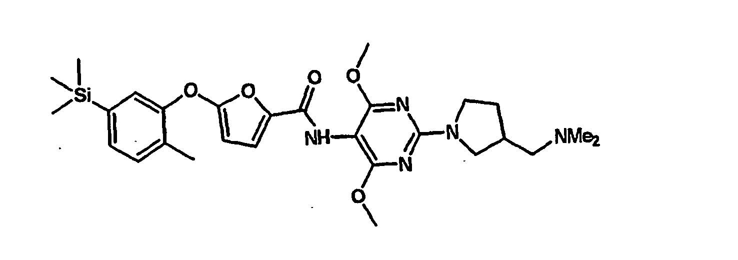

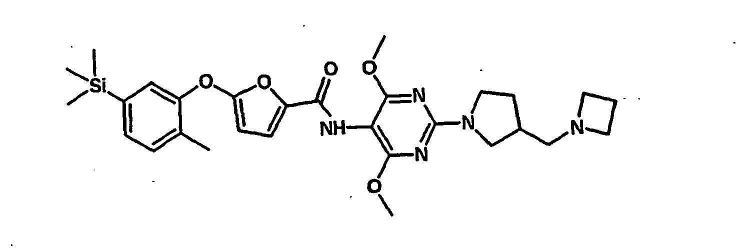

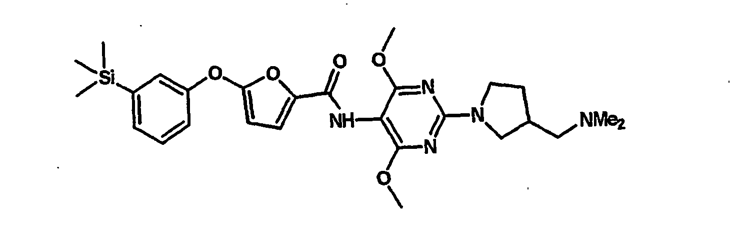

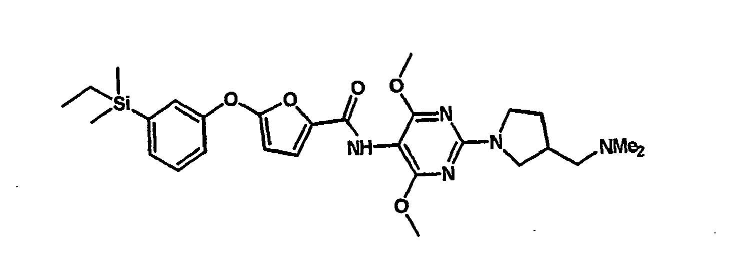

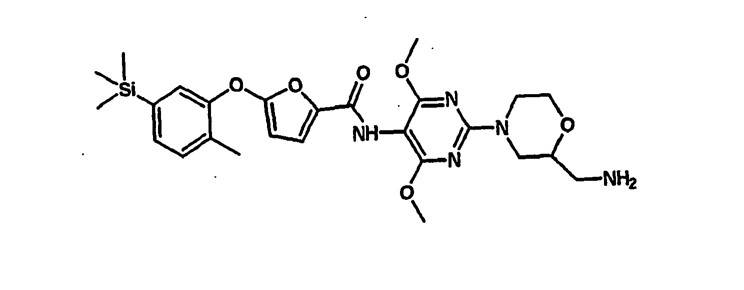

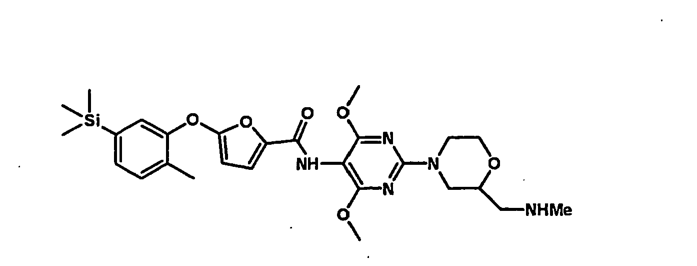

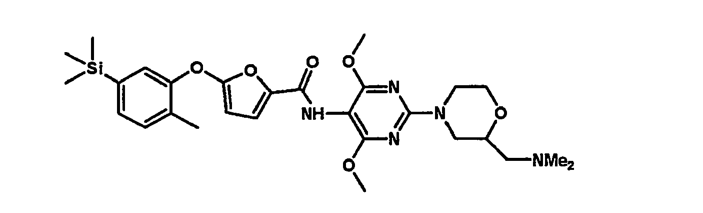

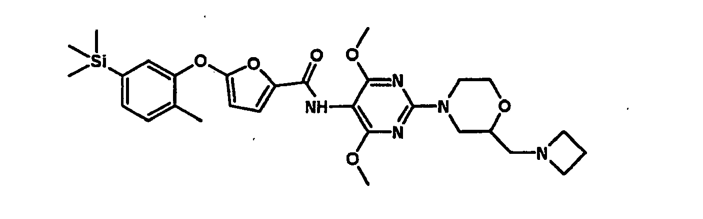

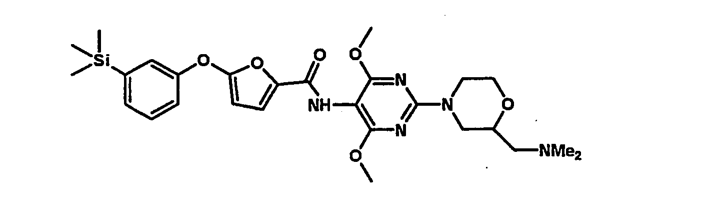

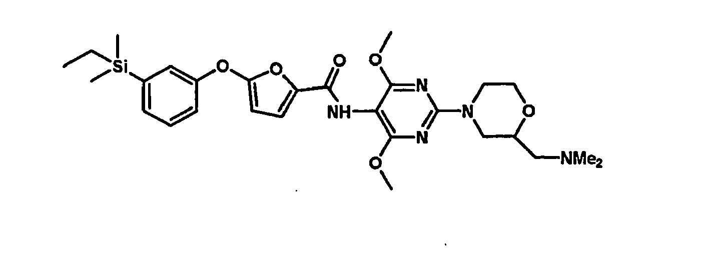

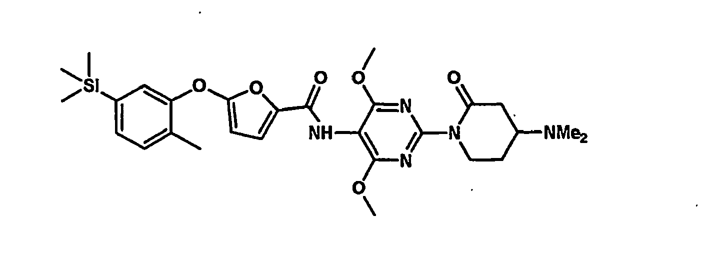

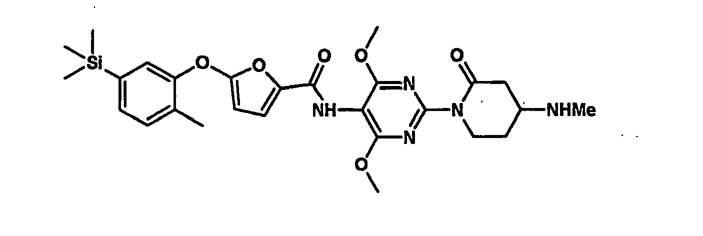

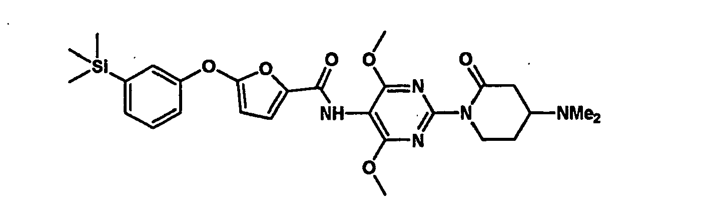

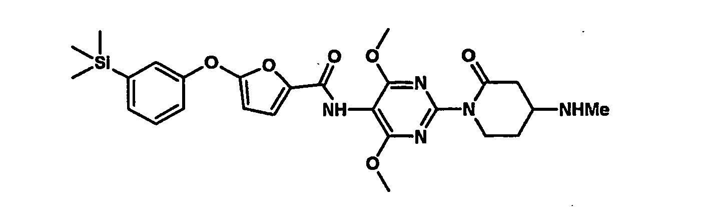

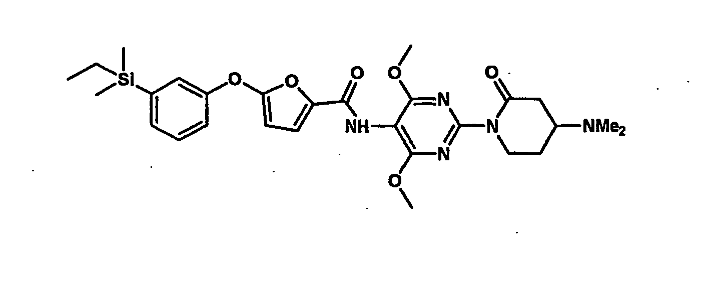

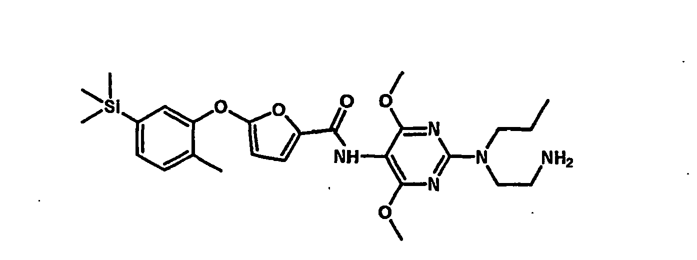

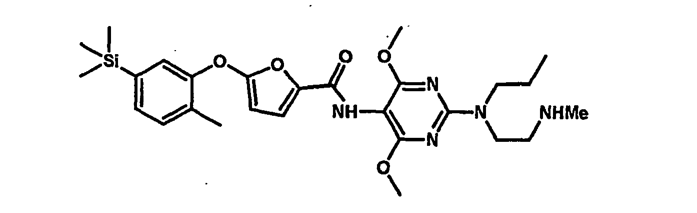

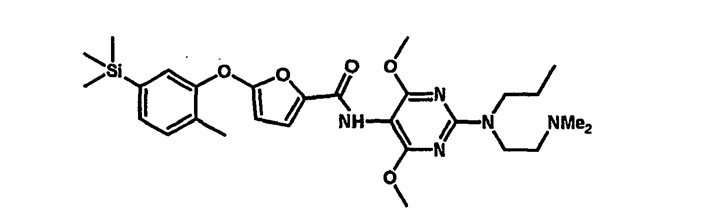

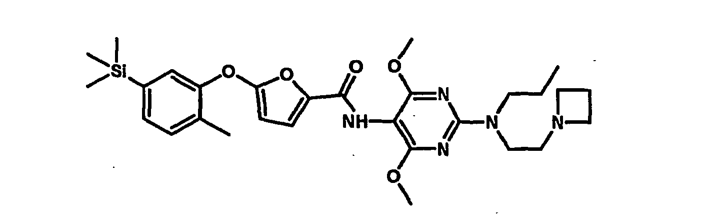

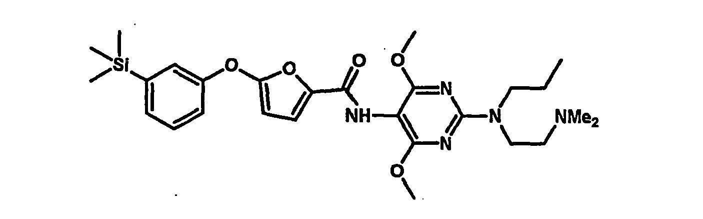

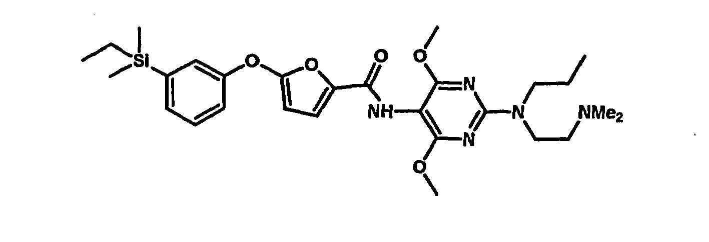

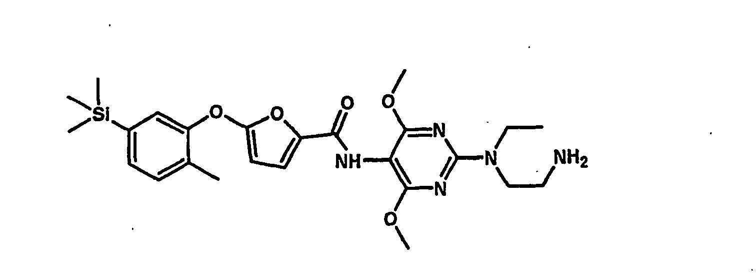

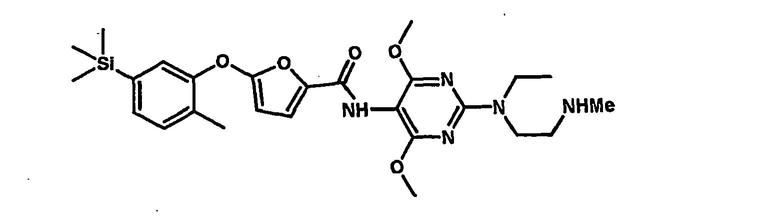

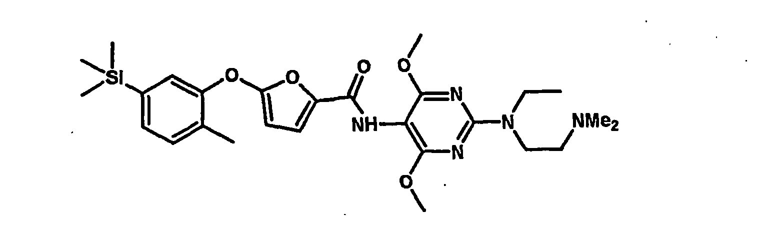

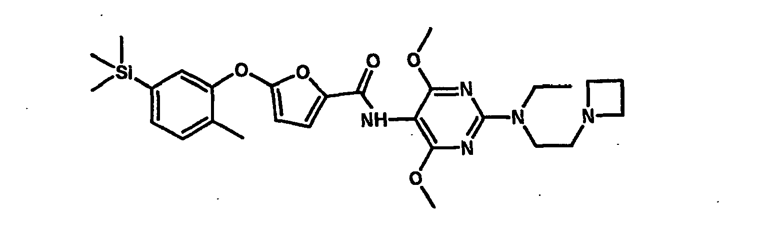

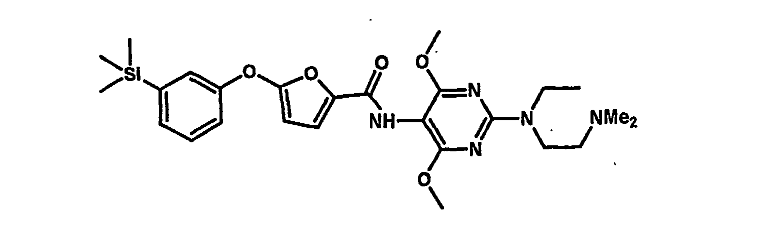

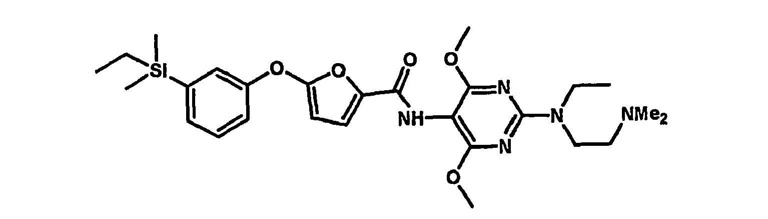

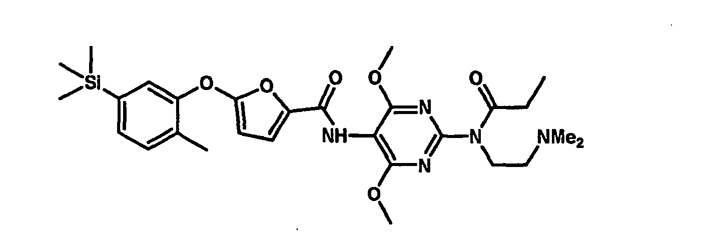

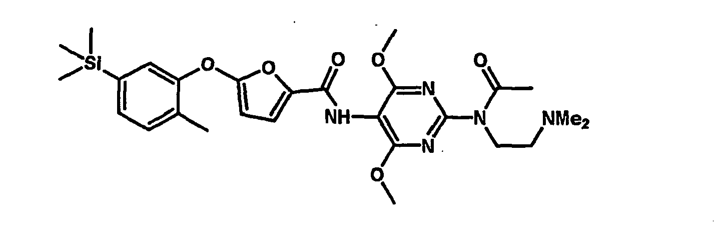

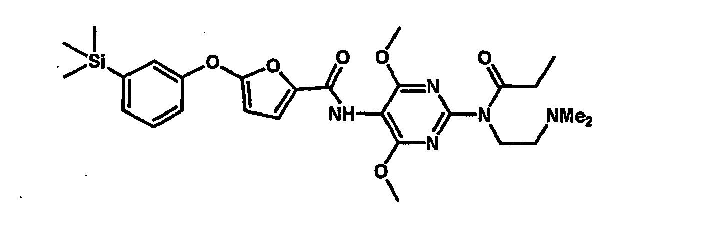

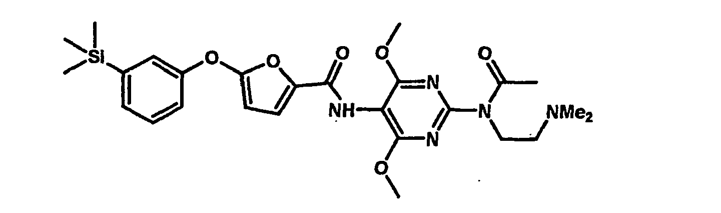

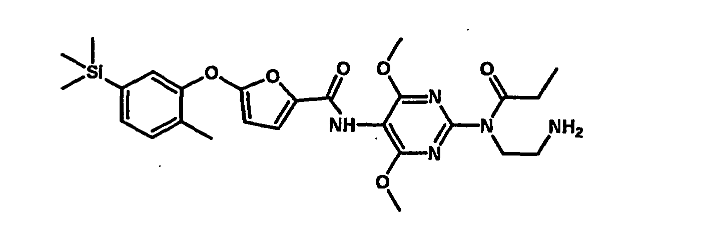

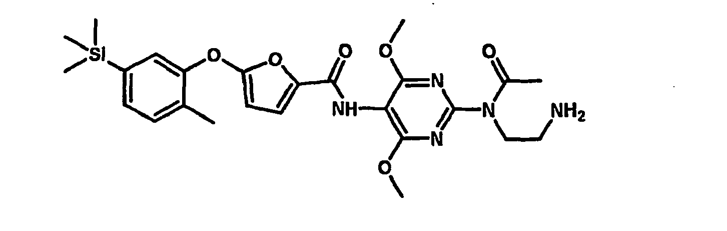

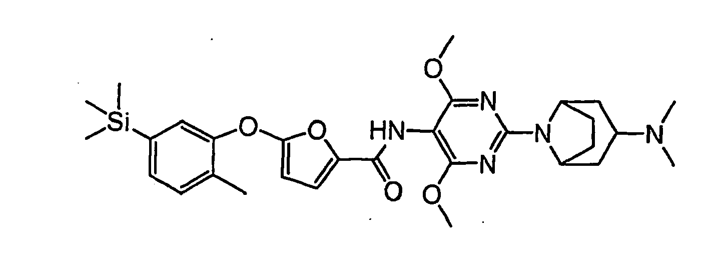

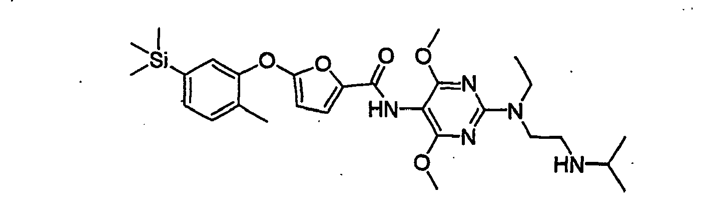

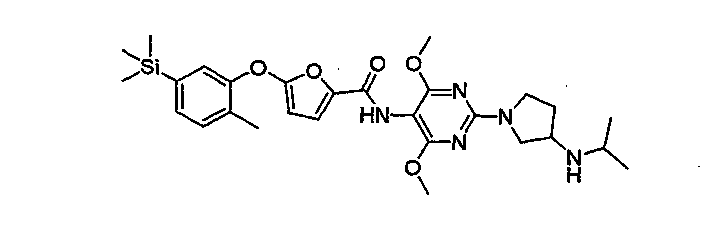

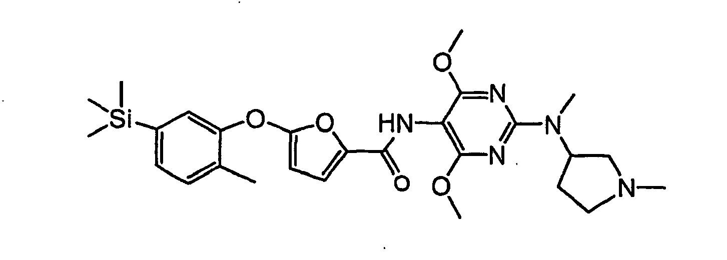

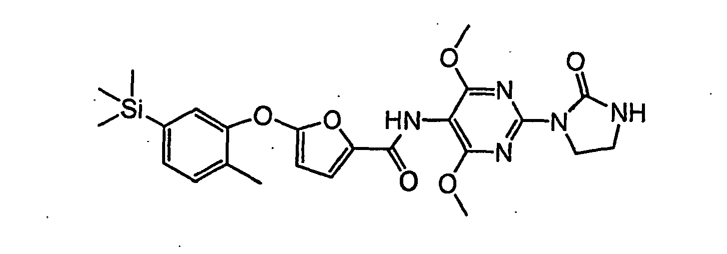

Compounds of the invention may be chiral. They may be in the form of a single enantiomer or diastereomer, or a racemate.

The compounds of the invention may be prepared in racemic form, or prepared in individual enantiomeric form by specific synthesis or resolution as will be appreciated in the art. The compounds may, for example, be resolved into their enantiomers by standard techniques, such as the formation of diastereomeric pairs by salt formation with an optically active acid followed by fractional crystallisation and regeneration of the free base. Alternatively, the enantiomers of the novel compounds may be separated by HPLC using a chiral column.

Some compounds of the formula may exist in different tautomeric forms, which also fall within the scope of the invention.

A compound of the invention may be in a protected amino, or protected hydroxy or protected carboxy form. The terms "protected amino", "protected hydroxy" and "protected carboy" as used herein refer to amino, hydroxy and carboxy groups which are protected in a manner familiar to those skilled in the art. For example, an amino group can be protected by a benzyloxycarbonyl, tert-butoxycarbonyl, acetyl or like group, or in the form of a phthalimido or like group. A carboxyl group can be protected in the form of a readily cleavable ester such as the methyl, ethyl, benzyl or tert-butyl ester.

Some compounds of the formula may exist in the form of solvates, for example hydrates, which also fall within the scope of the present invention.

Compounds of the invention may be in the form of pharmaceutically acceptable salts, for example, addition salts of inorganic or organic acids. Such inorganic acid addition salts include, for example, salts of hydrobromic acid, hydrochloric acid, nitric acid, phosphoric acid and sulphuric acid. Organic acid addition salts include, for example, salts of acetic acid, benzenesulphonic acid, benzoic acid, camphorsulphonic acid, citric acid, 2-(4-chlorophenoxy)-2-methylpropionic acid, 1,2-ethanedisulphonic acid, ethanesulphonic acid, ethylenediaminetetraacetic acid (EDTA), fumaric acid, glucoheptonic acid, gluconic acid, glutamic acid, N-glycolylarsanilic acid, 4-hexylresorcinol, hippuric acid, 2-(4-hydroxybenzoyl)benzoic acid, 1-hydroxy-2-naphthoic acid, 3-hydroxy-2-naphthoic acid, 2-hydroxyethanesulphonic acid, lactobionic acid, n-dodecyl sulphuric acid, maleic acid, malic acid, mandelic acid, methanesulphonic acid, methyl sulphuric acid, mucic acid, 2-naphthalenesulphonic acid, pamoic acid, pantothenic acid, phosphanilic acid ((4-aminophenyl)phosphonic acid), picric acid, salicylic acid, stearic acid, succinic acid, tannic acid, tartaric acid, terephthalic acid, p-toluenesulphonic acid, 10-undecenoic acid and the like.

It will be appreciated that such salts, provided that they are pharmaceutically acceptable, may be used in therapy. Such salts may be prepared by reacting the compound with a suitable acid in a conventional manner.

A compound of the invention may be prepared by any suitable method known in the art and/or by the following processes:

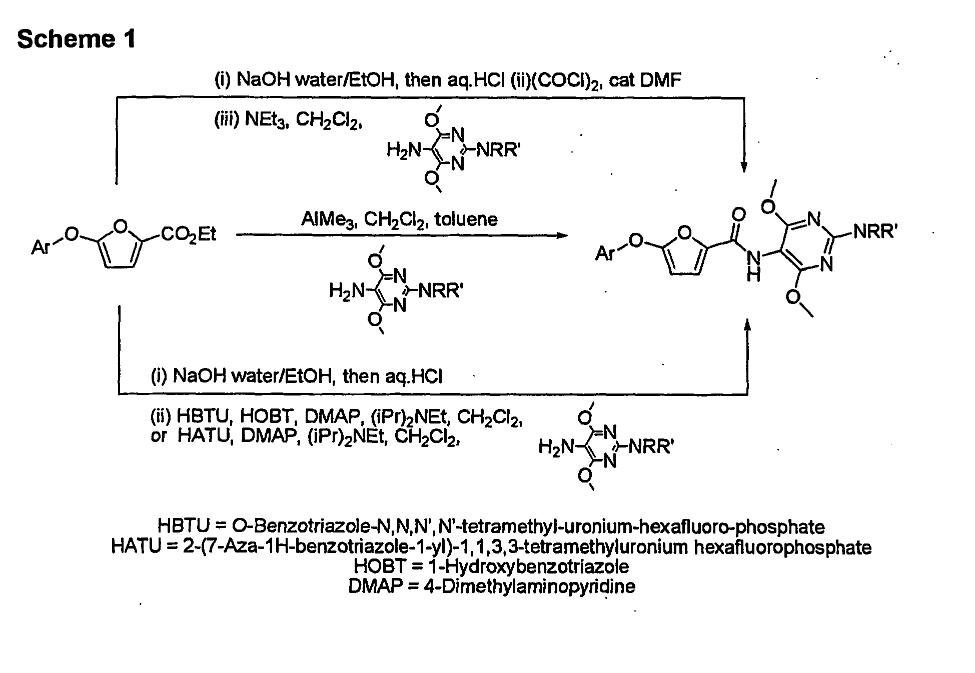

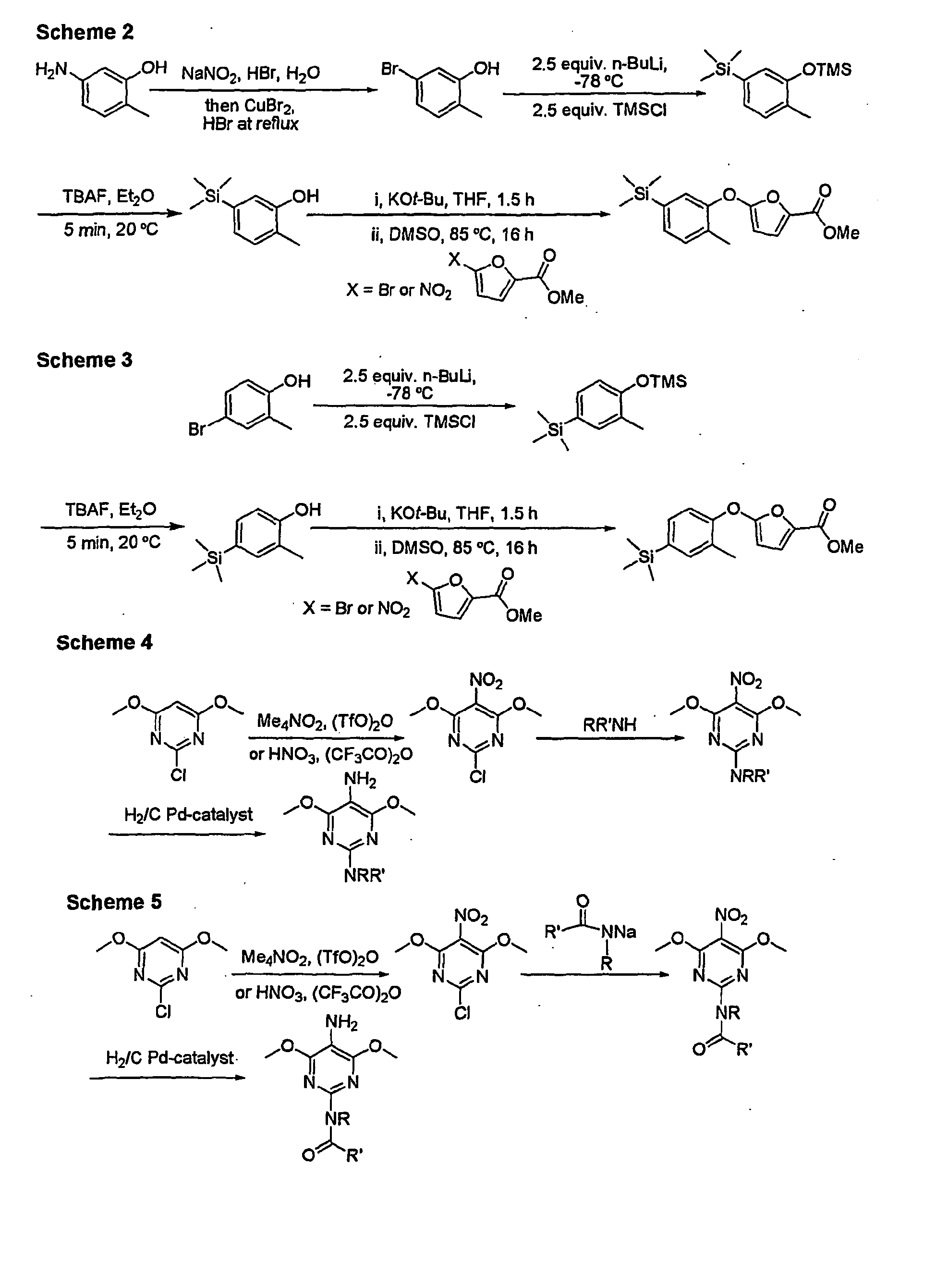

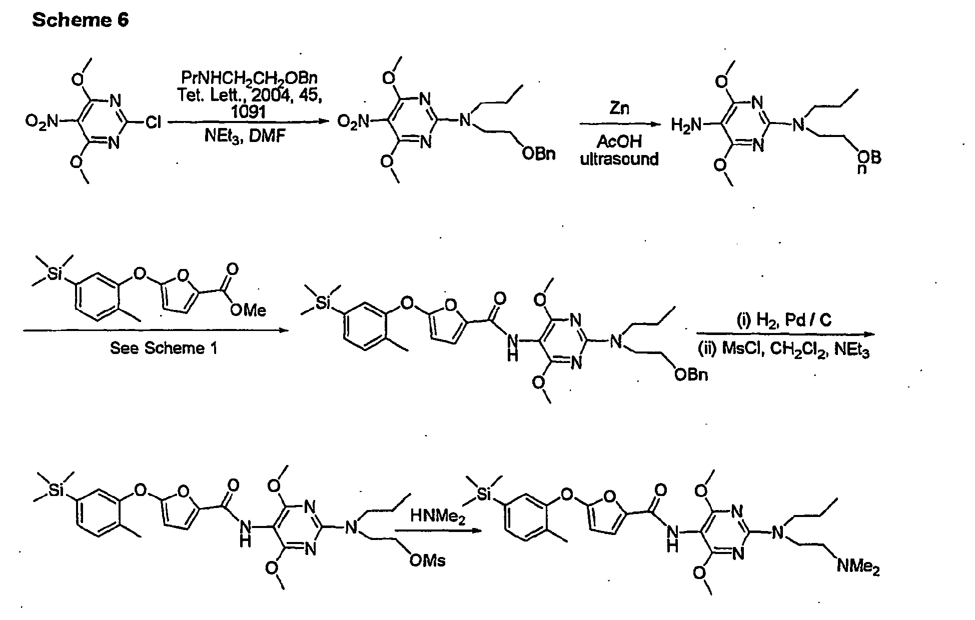

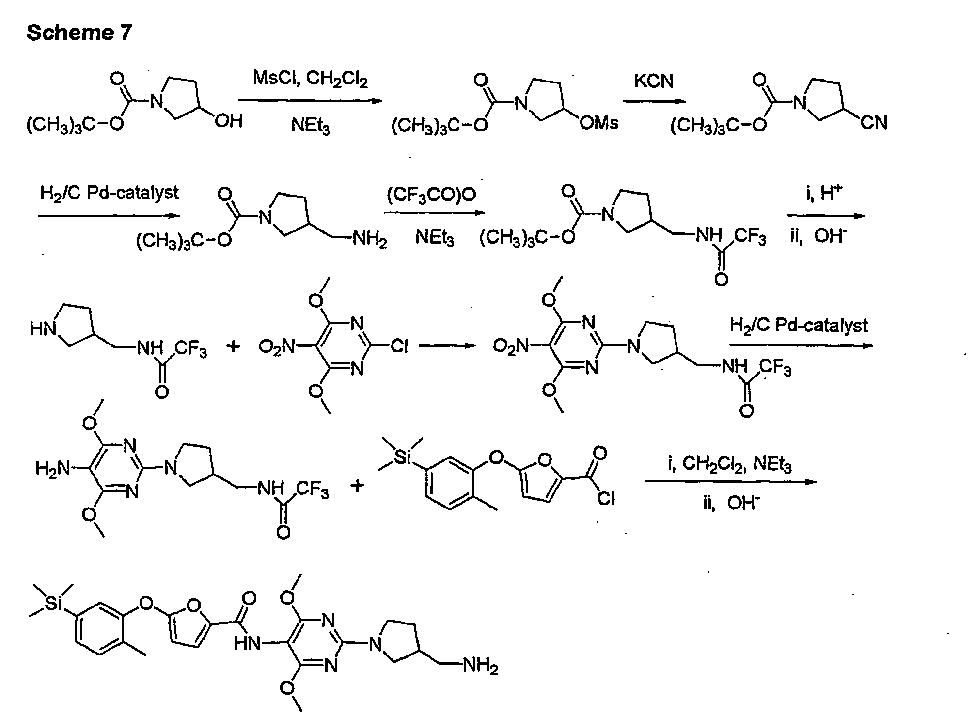

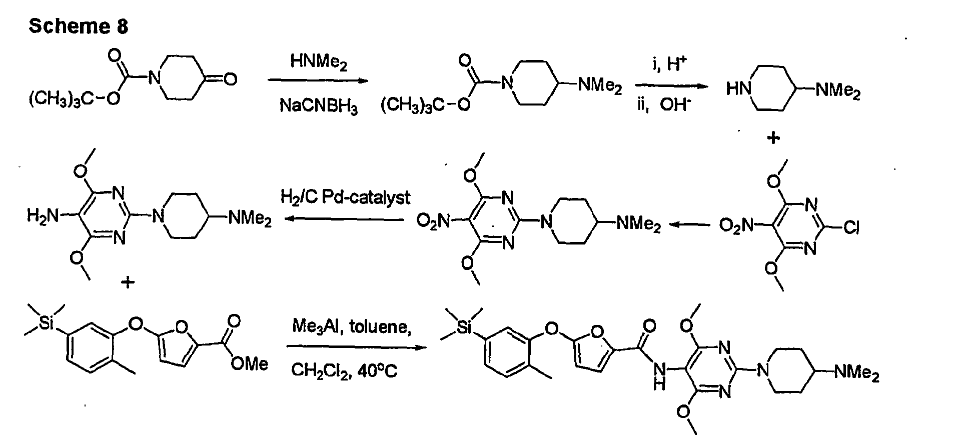

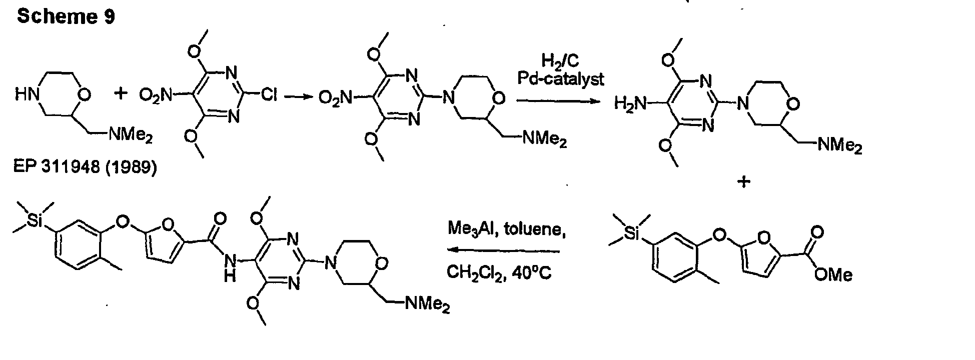

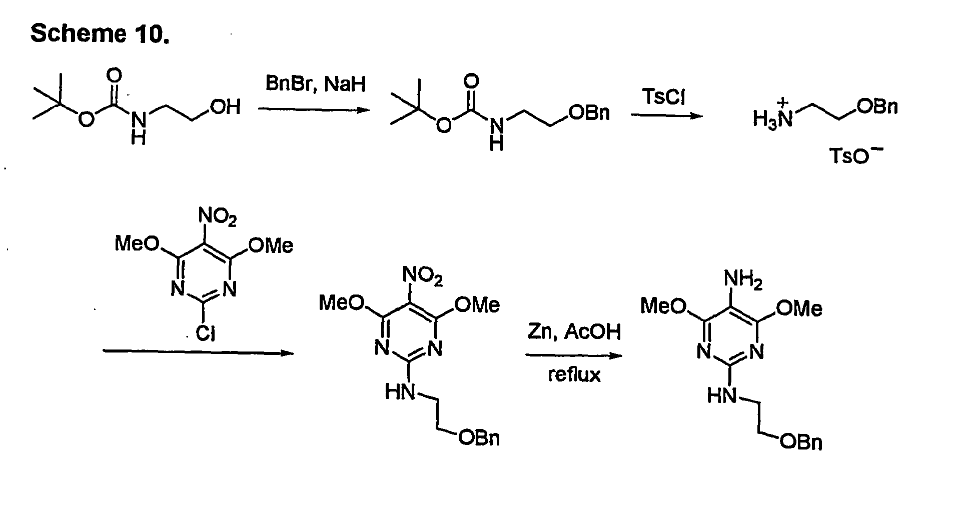

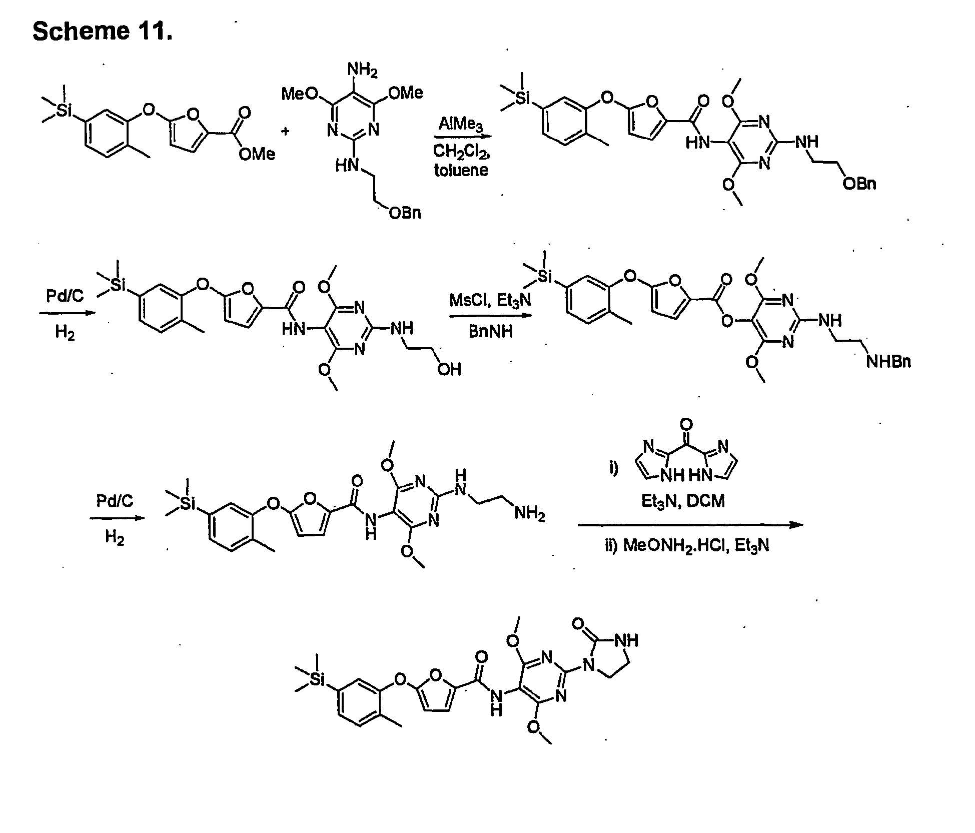

It will be understood that the processes detailed above are solely for the purpose of illustrating the invention and should not be construed as limiting. A process utilising similar or analogous reagents and/or conditions known to one skilled in the art may also be used to obtain a compound of the invention.

Any mixtures of final products or intermediates obtained can be separated on the basis of the physico-chemical differences of the constituents, in a known manner, into the pure final products or intermediates, for example by chromatography; distillation, fractional crystallisation, or by the formation of a salt if appropriate or possible under the circumstances.

The activity and selectivity of the compounds may be determined by any suitable assay known in the art.

The compounds of the invention may be used in the treatment of numerous ailments, conditions and diseases including, but not limited thereto, cancer, endometriosis, uterine myoma, an ovarian disease, a mammary cystic disease, prostatic hypertrophy, amenorrhea, precocious puberty, premenstrual syndrome, a sex-steroid-dependent pathophysiology, benign prostatic hyperplasia, Alzheimer's disease, HIV infection, AIDS and diseases caused by thyroid malfunction, or to arrest spermatogenesis.

The term 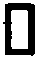 cancer 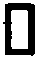 as used herein refers to any disease or condition characterised by uncontrolled, abnormal growth of cells and includes all known types of cancer, for example cancer of the bladder, breast, colon, brain, bone, head, blood, eye, neck, skin, lungs, ovaries, prostate and rectum; digestive, gastrointestinal, endometrial, hematological, AIDS-related, muscoskeletal, neurological and gynecological cancers; lympomas, melanomas and leukaemia.

In therapeutic use, the active compound may be administered orally, rectally, intra-vaginally, parenterally, by inhalation (pulmonary delivery), topically, ocularly, nasally, or to the buccal cavity. Oral administration is preferred. Thus, the therapeutic compositions of the present invention may take the form of any of the known pharmaceutical compositions for such methods of administration. The compositions may be formulated in a manner known to those skilled in the art so as to give a controlled release, for example rapid release or sustained release, of the compounds of the present invention. Pharmaceutically acceptable carriers suitable for use in such compositions are well known in the art. The compositions of the invention may contain 0.1-99% by weight of active compound. The compositions of the invention are generally prepared in unit dosage form. Preferably, a unit dose comprises the active ingredient in an amount of 1-500 mg. The excipients used in the preparation of these compositions are the excipients known in the art.

Appropriate dosage levels may be determined by any suitable method known to one skilled in the art. It will be understood, however, that the specific dose level for any particular patient will depend upon a variety of factors including the activity of the specific compound employed, the age, body weight, general health, sex, diet, time of administration, route of administration, rate of excretion, drug combination and the severity of the disease undergoing treatment.

Compositions for oral administration are preferred compositions of the invention and there are known pharmaceutical forms for such administration, for example tablets, capsules, granules, syrups and aqueous or oily suspensions. The pharmaceutical composition containing the active ingredient may be in a form suitable for oral use, for example, as tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions, and such compositions may contain one or more agents selected from the group consisting of sweetening agents, flavouring agents, colouring agents and preserving agents in order to provide pharmaceutically elegant and palatable preparations. Tablets contain the active ingredient in admixture with non-toxic pharmaceutically acceptable excipients which are suitable for the manufacture of tablets. These excipients may be, for example, inert diluents, such as calcium carbonate, sodium carbonate, lactose, calcium phosphate or sodium phosphate; granulating and disintegrating agents, for example corn starch or alginic acid; binding agents, for example starch gelatin, acacia, microcrystalline cellulose or polyvinyl pyrrolidone; and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed.

Formulations for oral use may also be presented as hard gelatin capsules wherein the active ingredient is mixed with an inert solid diluent, for example calcium carbonate, calcium phosphate or kaolin, or as soft gelatin capsules wherein the active ingredient is mixed with water or an oil medium, for example peanut oil, liquid paraffin or olive oil.

Aqueous suspensions contain the active materials in admixture with excipients suitable for the manufacture of aqueous suspensions. Such excipients are suspending agents, for example sodium carboxymethylcellulose, methylcellulose, hydroxypropylmethylcellulose, sodium alginate, polyvinyl pyrrolidone, gum tragacanth and gum acacia; dispersing or wetting agents may be a naturally occurring phosphatide, for example lecithin, or condensation products of an alkylene oxide with fatty acids, for example polyoxyethylene stearate, or condensation products of ethylene oxide with long-chain aliphatic alcohols, for example heptadecaethyleneoxycetanol, or condensation products of ethylene oxide with partial esters derived from fatty acids, for example polyoxyethylene sorbitan monooleate. The aqueous suspensions may also contain one or more preservatives, for example ethyl or n-propyl p-hydroxybenzoate, one or more colouring agents, one or more flavouring agents, and one or more sweetening agents, such as sucrose or saccharin.

Oily suspensions may be formulated by suspending the active ingredient in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents, such as those set forth above, and flavouring agents may be added to provide a palatable oral preparation. These compositions may be preserved by the addition of an antioxidant such as ascorbic acid.

Dispersible powders and granules suitable for preparation of an aqueous suspension by the addition of water provide the active ingredient in admixture with a dispersing or wetting agent, suspending agent and one or more preservatives. Suitable sweetening, flavouring and colouring agents may also be present.

The pharmaceutical compositions of the invention may also be in the form of oil-in-water emulsions. The oily phase may be a vegetable oil, for example olive oil or arachis oil, or a mineral oil, for example liquid paraffin, or mixtures of these. Suitable emulsifying agents may be naturally occurring gums, for example gum acacia or gum tragacanth, naturally occurring phosphatides, for example soya bean, lecithin, and esters or partial esters derived from fatty acids and hexitol anhydrides, for example sorbitan monooleate and condensation products of the said partial esters with ethylene oxide, for example polyoxyethylene sorbitan monooleate. The emulsions may also contain sweetening and flavouring agents.

Syrups and elixirs may be formulated with sweetening agents, for example glycerol, propylene glycol, sorbitol or sucrose. Such formulations may also contain a demulcent, a preservative and flavouring and colouring agents. The pharmaceutical compositions may be in the form of a sterile injectable aqueous or oleagenous suspension. This suspension may be formulated according to the known art using those suitable dispersing or wetting agents and suspending agents which have been mentioned above. The sterile injectable preparation may also be in a sterile injectable solution or suspension in a non-toxic parenterally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or diglycerides. In addition, fatty acids such as oleic acid, find use in the preparation of injectables.

The compounds of the invention may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials are cocoa butter and polyethylene glycols.

Compositions for topical administration are also suitable for use in the invention. The pharmaceutically active compound may be dispersed in a pharmaceutically acceptable cream, ointment or gel. A suitable cream may be prepared by incorporating the active compound in a topical vehicle such as light liquid paraffin, dispersed in a aqueous medium using surfactants. An ointment may be prepared by mixing the active compound with a topical vehicle such as a mineral oil or wax. A gel may be prepared by mixing the active compound with a topical vehicle comprising a gelling agent. Topically administrable compositions may also comprise a matrix in which the pharmaceutically active compounds of the present invention are dispersed so that the compounds are held in contact with the skin in order to administer the compounds transdermally.

The following Examples illustrate the invention.

In the Examples and Intermediates, all syntheses were carried out under dry nitrogen. Tetrahydrofuran (THF), diethyl ether, dichloromethane, toluene and m-xylene were dried and purified according to standard procedures and stored under nitrogen (as described in The Purification of Laboratory Chemicals, D. D. Perrin, W. L. F. Armarego, D. R. Perrin and C. Chai, Butterworth-Heinemann, 2003). Light petroleum refers to the fraction with b. p. 40-60 °C. DMSO refers to dimethylsulfoxide. Thin layer chromatography (TLC) was performed on silica (SiO₂) plates. ¹H NMR spectra were generated at 400 MHz in CDCl₃ unless otherwise stated. Mass spectral data were generated on a Waters ZQ instrument.

### Intermediate 1: 4,6-Dimethoxy-N-methyl-N-(1-methylpyrrolidin-3-yl)-5-nitropyrimidin-2-amine

*N*,1-Dimethylpyrrolidin-3-amine (5 g, 44.2 mmol) was dissolved in ethanol (75 mL) at 0 °C and the solution was cooled in an ice-bath. 2-Chloro-4,6-dimethoxy-5-nitropyrimidine was added portion-wise over 10 minutes and the solution was warmed to room temperature and stirred overnight. The ethanol was removed under reduced pressure and the residue was partitioned between dichloromethane and saturated aqueous sodium bicarbonate solution. The layers were separated and the aqueous portion was extracted with dichloromethane, the combined organics layers were then dried and the solvent was removed under reduced pressure to give solid which was used in further synthesis without purification (2.69 g, 87 %), R_{f} (EtOAc: Petrol = 1:4) 0.76; δ_{H} 1.81 (2 H, m), 2.43 (2 H, m), 2.40 (3 H, s), 2.65-2.79 (3 H, m), 3.15 (3 H, s) and 4.01 (6 H, s); MS (ES⁺) m/z 298 (MH⁺).

### Intermediate 2: 4,6-Dimethoxy-N²-methyl-N²-(1-methylpyrrolidin-3-yl)-pyrimidin-2,5-diamine

4,6-Dimethoxy-N-methyl-N-(1-methylpyrrolidin-3-yl)-5-nitropyrimidin-2-amine (**Intermediate 1**: 2.26g, 10 mmol) was dissolved in ethanol (75 mL). Pd-C (500 mg, 10% Pd on carbon) was added to this mixture under hydrogen (balloon) at room temperature. The resulting solution was stirred for 24 h at room temperature before being filtered through a short pad of celite and the filtrate was evaporated under reduced pressure to give the title compound (1.98 g, 99 %); δ_{H} 1.89 (1 H, m), 2.39 (3 H, s), 2.59 (3 H, m), 2.75 (3 H, m), 3.03 (3 H, s) and 3.93 (6 H, s). MS (ES⁺) m/z 268 (MH⁺).

### Intermediate 3: 1-(4,6-dimethoxy-5-nitropyrimidin-2-yl)-N,N-dimethylpyrrolidin-3-amine

N,N-Dimethylpyrrolidin-3-amine (5 g, 39.0 mmol) was dissolved in ethanol (75 mL) at 0 °C and the solution was cooled in an ice-bath. 2-Chloro-4,6-dimethoxy-5-nitropyrimidine was added portion-wise over 10 minutes and the solution was warmed to room temperature and stirred overnight. The ethanol was removed under reduced pressure and the residue was partitioned between dichloromethane and saturated aqueous sodium bicarbonate solution. The layers were separated and the aqueous layer was extracted with dichloromethane, the combined organics layers were then dried and the solvent was removed under reduced pressure to give solid (2.69 g, 87 %), R_{f} (EtOAc: Petrol = 1:4) 0.7; δ_{H} 1.97 (1 H, m), 2.21 (2 H, m), 2.33 (6 H, s), 2.82 (1 H, m), 3.34 (1 H, dd, J = 8.6 and 11.6), 3.52 (1 H, ddd, J = 7, 10.6 and 12.1), 3.87 (1 H, m), 3.93 (1 H, dd, J = 7 and 11.6), 4.01 (3 H, s) and 4.02 (3 H, s); MS (ES⁺) m/z 298(MH⁺)

### Intermediate 4: 2-(3-(Dimethylamino)pyrrolidin-1-yl)-4,6-dimethoxypyrimidin-5-amine

1-(4,6-dimethoxy-5-nitropyrimidin-2-yl)-N,N-dimethylpyrrolidin-3-amine (**Intermediate 3;** 2.0g, 10 mmol) was dissolved in ethanol (75 mL). Pd-C (500 mg, 10 % Pd on carbon) was added to this mixture under hydrogen balloon at room temperature and the reaction was stirred for 24 h at room temperature. The resulting mixture was filtered through a short pad of celite and the filtrate was evaporated under reduced pressure to afford the title compound (1.8 g, 98 %); δ_{H} 1.85 (1 H, m), 2.16 (1 H, m), 2.32 (6 H,s), 2.76 (1 H, m), 3.23 (1 H, dd, J = 7 and 10.6), 3.43 (1 H, m), 3.74 (1 H, m), 3.86 (1 H, dd, J = 7 and 10.6) and 3.96 (6 H, s); MS (ES⁺) m/z 268(MH⁺).

### Intermediate 5: tert-butyl 2-(benzyloxy)ethylcarbamate

To a cooled (0 °C) solution of *tert*-butyl 2-hydroxyethylcarbamate (25 g, 0.16 mol) in THF (250 mL) was added sodium hydride (7.74 g, 1.2 mol. equiv.) portion-wise. The reaction was allowed to stir at this temperature for 1.5 h before the cooling bath was removed and stirring was continued for a further 1 h. Benzyl bromide (23 mL, 1.2 mol. equiv.) was added drop-wise and the reaction was allowed to stir overnight before water (250 mL) was added and the mixture was extracted into diethyl ether (3 x 250 mL). The combined organic extracts were dried (MgSO₄) and the volatiles were removed *in vacuo*.

The crude product was purified by column chromatography (silica gel, 4:1 light petroleum-ether) to afford the desired product (19.85 g, 54 %)

### Intermediate 6: 2-(benzyloxy)ethanamine para-toluenesulfonic acid salt

To a solution of *tert*-butyl 2-(benzyloxy)ethylcarbamate (**Intermediate 5;** 18.0 g, 75.9 mmol) in acetonitrile (180 mL) was added para-toluene sulfonic acid (15.88 g, 1.1 mol. equiv.) in acetonitrile (180 mL). The mixture was heated to reflux for 2 h before being concentrated *in vacuo* to afford the crude product (27.34 g, 111 %) which was used directly in the next reaction without further purification.

### Intermediate 7: N-(2-(benzyloxy)ethyl)-4,-dimethoxy-5-nitropyrimidin-2-amine

To a solution of 2-(benzyloxy)ethanamine *para*-toluenesulfonic acid salt (**Intermediate 6;** 5.0 g, 15.46 mmol.) in dry *N,N*-dimethylformamide (50 mL) was added triethylamine (4.3 mL, 2.0 mol. equiv.) and 2-chloro-4,6-dimethoxy-5-nitropyrimidine (3.39 g, 15.46 mmol.). The mixture was stirred overnight at room temperature before being quenched with water (250 mL) and then extracted with ethyl acetate (1 x 250 mL then 2 x 100 mL). The combined organic extracts were dried (MgSO₄) and evaporated *in vacuo* to afford a yellow oil. The crude product was purified by column chromatography (silica gel, 4:1 to 1:1 light petroleum-ethyl acetate) to afford the title product (4.37 g, 85 %).

### Intermediate 8: N-(2-(benzyloxy)ethyl)-4,6-dimethoxypyrimidine-2,5-diamine

To a solution of N-(2-(benzyloxy)ethyl)-4,6-dimethoxy-5-nitropyrimidin-2-amine (**Intermediate 7;** 15.6 g, 46.66 mmol.) in acetic acid (150 mL) was added powdered zinc metal (15.24 g, 5 mol. equiv.) and the mixture activated using ultrasound for 6 h. A further portion of zinc (15.24 g) was added to the reaction and sonication was continued for a further 16 h. The volatiles were removed *in vacuo* and the residue taken up into ethyl acetate (500 mL) which was washed with brine (250 mL) and then with saturated aqueous sodium bicarbonate solution (400 mL). The organic fraction was then dried (MgSO₄) and evaporated under reduced pressure to afford the desired material (100 %) as a red oil which was used without further purification.

### Intermediate 9: N-(2-(2-(benzyloxy)ethylamino)-4,6-dimethoxypyrimidin-5-yl)-5-(2-methyl-5-(trimethylsilyl)phenoxy)furan-2-carboxamide

A solution of trimethylaluminium (12.4 mmol, 6.2 mL of 2.0 M solution) in toluene was added drop-wise at -30 °C within 10 minutes to N-(2-(benzyloxy)ethyl)-4,6-dimethoxypyrimidine-2,5-diamine (**Intermediate 8**; 1.89 g, 6.2 mmol) in toluene (5 mL). The reaction was stirred and allowed to warm to -20 °C over 30 min and then to room temperature over a further 30 min. This solution was then added to a solution of methyl 5-(2-methyl-5-(trimethylsilyl)phenoxy)furan-2-carboxylate (prepared according to the procedure in WO/050050442 A1, 1.89 g, 6.2 mmol) in dichloromethane (15 mL) at 0 °C. This mixture was allowed to warm to room temperature and stirred for a further 16 h before being treated with saturated aqueous ammonium acetate solution drop-wise until effervescence ceased The suspension was filtered through Celite, washing with several portions of ethyl acetate. The filtrates were washed with water and then dried (MgSO₄) before being concentrated *in vacuo* and purified by column chromatography (silica gel, 7:3 to 1:1 light petroleum-ethyl acetate) to afford the desired material (2.1 g, 58 %).

### Intermediate 10: N-(2-(2-hydroxyethylamino)-4,6-dimethoxypyrimidin-5-yl)-5-(2-methyl-5-(trimethylsilyl)phenoxy)furan-2-carboxamide

To a solution of N-(2-(2-(benzyloxy)ethylamino)-4,6-dimethoxypyrimidin-5-yl)-5-(2-methyl-5-(trimethylsilyl)phenoxy)furan-2-carboxamide (**Intermediate 9;** 1.05 g, 1.8 mmol.) in ethyl alcohol (30 mL) was added palladium on carbon (10 % by weight, 100 mg) and the mixture was stirred under a balloon of hydrogen for 3 h before being purged with nitrogen. The suspension was filtered through Celite and concentrated *in vacuo* to afford the crude product (690 mg, 78 %) which was used directly without further purification.

### Intermediate 11: 2-(4,6-dimethoxy-5-(5-(2-methyl-5-(trimethylsilyl)phenoxy)furan-2-carboxamido)pyrimidin-2-ylamino)ethyl methanesulfonate

To a solution of N-(2-(2-hydroxyethylamino)-4,6-dimethoxypyrimidin-5-yl)-5-(2-methyl-5-(trimethylsilyl)phenoxy)furan-2-carboxamide (**Intermediate 10:** 111 mg, 0.23 mmol.) in dichloromethane (2 mL) at 0 °C was added triethylamine (64 µL, 0.46 mmol.) and then methanesulfonyl chloride (35 µL, 0.46 mmol.). The reaction was allowed to stir for 5 min. before being quenched with water. The resulting mixture was extracted into dichloromethane (2 x 10 mL) which was washed with saturated aqueous sodium bicarbonate solution, dried (MgSO₄) and concentrated *in vacuo* to afford the product which was used directly without further purification.

### Intermediate 12: N-(2-(2-(benzylamino)ethylamino)-4,6-dimethoxypyrimidin-5-yl)-5-(2-methyl-5-(trimethylsilyl)phenoxy)furan-2-carboxamide

To a solution of 2-(4,6-dimethoxy-5-(5-(2-methyl-5-(trimethylsilyl)phenoxy)-furan-2-carboxamido)pyrimidin-2-ylamino)ethyl methanesulfonate (**Intermediate 11**: 2.6 mmol.) in THF (15 mL) was added benzylamine (2.0 mL) and the mixture was allowed to stir at 55 ° for 18 h. A further portion of benzylamine (1 mL) was added and the reaction was allowed to stir at 55 °C for a further 20 h. Upon cooling, the mixture was extracted into ethyl acetate (2 x 25 mL) and the combined organic extracts were dried (MgSO₄) and concentrated *in vacuo* to afford the crude product. This was purified by column chromatography (silica gel; 19:1 dichloromethane-methyl alcohol) to afford the pure title compound (418 mg, 28 %).

### Intermediate 13: N-(2-(2-aminoethylamino)-4,6-dimethoxypyrimidin-5-yl)-5-(2-methyl-5-(trimethylsilyl)phenoxy)furan-2-carboxamide

To a solution of N-(2-(2-(benzylamino)ethylamino)-4,6-dimethoxypyrimidin-5-yl)-5-(2-methyl-5-(trimethylsilyl)phenoxy)furan-2-carboxamide (**Intermediate 12**; 39 mg, 6.8 x 10⁻⁵ moles) was taken up into ethyl alcohol (2 mL) and treated with 10 % palladium on carbon (5 mg) before being allowed to stir at room temperature under an atmosphere of hydrogen for 4 h. The resulting mixture was filtered through Celite and the filtrate concentrated *in vacuo* to afford the crude product which was purified by column chromatography (silica gel, 9:1-dichloromethane-methyl alcohol containing ca. 1 % 0.88 aqueous ammonia solution) to afford the title compound (16 mg, 48 %).

### Examples 1: N-(4,6-Dimethoxy-2-(methyl(1-methylpyrrolidin-3-yl)amino)pyrimidin-5-yl)-5-(2-methyl-5-(trimethylsilyl)phenoxy)furan-2-carboxamide

A solution of trimethylaluminium (4.5 mmol, 3 mL of 1.5 M solution) in toluene (10 mL) was added drop-wise at -30 °C within 10 minutes to a stirred suspension of 4,6-dimethoxy-*N*²-methyl-*N*²-(1-methylpyrrolidin-3-yl)-pyrimidin-2,5-diamine (**Intermediate 2**; 0.4 g, 1.5 mmol) in toluene (5 mL). The stirred mixture was warmed to -20 °C over 30 minutes and the resulting solution was then added drop-wise to a stirred solution of methyl 5-(2-methyl-5-(trimethylsilyl)phenoxy)furan-2-carboxylate (prepared according to the procedure in WO/ 050050442 A1, 0.45 g, 1.5 mmol) in dichloromethane (15 mL) at 0 °C. The resulting mixture was stirred at room temperature for 30 minutes and then at 40 °C for 3 days. The reaction mixture was quenched with ammonium acetate cautiously and extracted with ether (3 × 30 mL). The organic layer was dried (MgSO₄), filtered and the filtrate was evaporated under reduced pressure. The residue was chromatographed to afford the title compound (215 mg, 27 %) as white solid, mp 74 °C (CH₂Cl₂); R_{f} (MeOH: CH₂Cl₂ = 3:7) 0.8; δ_{H} 1.88 (2 H, m), 2.18 (1 H, m), 2.32 (3 H, s), 2.34 (6 H, s), 2.79 (1 H, m), 3.28 (1 H, dd, J = 8.6 and 11.1), 3.49 (1 H, dd, J = 7 and 10), 3.81 (1 H, m), 3.92 (6 H, s), 5.34 (1 H, d, J = 3.5), 7.01 (1 H, s), 7.13 (1 H, d, J = 3.5), 7.19 (1 H, s) and 7.28 (2 H, m); δ_{C} (CDCl₃, 100 MHz) -1.1, 15.8, 30.1, 44.3, 45.6, 50.7, 53.4, 53.7, 65.4, 88.2, 90.1, 117.3, 123.0, 129.6, 130.3, 131.3, 139.1, 140.4, 152.9, 157.0, 157.3, 158.9 and 166.1; MS (ES⁺) m/z 540 (MH⁺).

### Example 2: N-(2-(3-(dimethylamino)pyrrolidin-1-yl)-4,6-dimethoxypyrimidin-5-yl)-5-(2-methyl-5-(trimethylsilyl)phenoxy)furan-2-carboxamide.

A solution of trimethylaluminium (4.5 mmol, 3 mL of 1.5 M) in toluene (10mL) was added drop-wise at -30 °C within 10 minutes to a stirred suspension of 2-(3-(dimethylamino)pyrrolidin-1-yl)-4,6-dimethoxypyrimidin-5-amine (**Intermediate 4**; 400 mg, 1.5 mmol) in toluene (5mL). The stirred mixture was warmed to -20 °C over 30 minutes and then added drop-wise to a stirred solution of methyl 5-(2-methyl-5-(trimethylsilyl)phenoxy)furan-2-carboxylate (prepared according to the procedure in WO/050050442 A1, 456 mg, 1.5 mmol) in dichloromethane (15 mL) at 0 °C. The resulting mixture was stirred at room temperature for 30 minutes and then at 40 °C for 3 days. The reaction mixture was quenched with ammonium acetate cautiously and extracted with ether (3 x 30 mL). The organic layer was dried (MgSO₄), filtered, and the filtrate was evaporated under reduced pressure. The residue was chromatographed to give the title compound (175 mg, 22 %), *R_{f}* (MeOH: CH₂Cl₂ = 3:7) 0.46; δ_{H} 1.91 (1 H, m), 2.26 (1 H, m), 2.33 (3 H, s), 2.38 (3 H, s), 2.71 (3 H, m), 2.83 (1 H, dd, m), 3.1 (3 H, s), 3.31 (3 H, s), 3.92 (6 H, s), 5.42 (1 H, d, J = 3.5), 5.6 (1 H, m), 7.12 (1 H, d, J = 3.5), 7.2 (1 H, m) and 7.35 (2 H, m); δ_{C} (CDCl₃, 100 MHz) 0.01, 17.1, 30.6, 31.1, 43.4, 56.8, 57.5, 60.4, 89.6, 91.5, 118.5, 124.6, 131.2, 132.0, 132.8, 140.6, 141.9, 154.4, 158.3, 160.4, 160.6 and 167.6; MS (ES⁺) m/z 540 (MH⁺)

### Example 3: N-(4,6-dimethoxy-2-(2-oxoimidazolidin-1-yl)pyrimidin-5-yl)-5-(2-methyl-5-(trimethylsilyl)phenoxy)furan-2-carboxamide

To a solution of N-(2-(2-aminoethylamino)-4,6-dimethoxypyrimidin-5-yl)-5-(2-methyl-5-(trimethylsilyl)phenoxy)furan-2-carboxamide (**Intermediate 13;** 160 mg, 0.33 mmol) in dichloromethane (10 mL) at 0 °C was added triethylamine (55 µL, 0.40 mmol) and carbonyldiimidazole (64 mg, 0.40 mmol). The reaction was allowed to warm to room temperature and stirred for 3 hours. O-methylhydroxylammonium chloride (234 mg, 2.8 mmol) and triethylamine (0.39 mL, 2.8 mmol) were added and the reaction stirred at room temperature for 42 hours. Water (5 mL) was added and the layers separated. The organic phase was washed with saturated aqueous sodium bicarbonate (5 mL), dried (MgSO₄) and concentrated under reduced pressure to give the crude product. Column chromatography (SiO₂; 98:2 dichloromethane/methanol) and recrystallisation from 2-propanol yielded the title compound as white crystals (29 mg, 17 %). *R*_{f} (MeOH: CH₂Cl₂ = 1:9) 0.36; δ_{H} 0.26 (9 H, s), 2.34 (3 H, s), 3.51-3.58 (2 H, t), 4.04 (6 H, s), 4.14 (2 H, t), 4.72 (1 H, s), 5.34 (1 H, s), 7.08 (1 H, s), 7.13 (1 H, s), 7.19 (1 H, s) and 7.26-7.31 (2 H, m); δ_{C} (CDCl₃, 100 MHz) -1.17, 15.9, 36.9, 44.7, 54.5, 88.3, 95.9, 117.3, 123.1, 129.6, 130.5, 131.4, 138.7, 140.5, 152.9, 154.1, 156.4, 157.8, 159.2 and 160.1; MS (ES⁺) m/z 512 (MH⁺).

### Example 4: Human GnRH receptor functional assay.

GnRH-receptor antagonists may be functionally assessed by measurement of change in intracellular calcium levels induced by Gαq mediated increase in IP3 levels. The ability of compounds to block the intracellular release of calcium by GnRH in CHO-K1 cells expressing human GnRH receptors is determined as a measure of the compound's antagonist activity *in vitro*. Approximately 30,000 cells per assay well (half well 96 well assay plate - Corning) are seeded in normal culture medium. Twenty four hours after seeding the cells are loaded with a calcium sensitive fluorescent dye by replacing the culture medium with assay buffer (1 x Hanks buffered saline, 25 mM HEPES, 0.1 % w/v fatty acid free BSA, pH7.4) containing 2.5 mM probenecid and 1 x Calcium Plus reagent (Molecular) Devices. Cells are incubated at 37 °C for 1 hour to allow for dye uptake. To test for antagonist activity, compounds at a concentration range between 0.1 nM - 3.2 µM are added to the assay wells and allowed to incubate 20 minutes prior to stimulation with GnRH. After incubation with test compounds the assay plate is placed in a Flexstation II (Molecular Devices) and GnRH is added at the determined EC₈₀ concentration (final). Ligand-dependent changes in intracellular calcium levels are determined by measuring changes in fluorescence of the dye at 525 nM following excitation at 485 nM. Percentage inhibition curves are plotted using 4-parameter fit algorithm and IC₅₀ values calculated for each compound.

| **Example Number** | **Compound Name** | **Human GnRH IC₅₀ (nM)** |
|---|---|---|
| **2** | N-(2-(3-(dimethylamino)pyrrolidin-1-yl)-4,6-dimethoxypyrimidin-5-yl)-5-(2-methyl-5-(trimethylsilyl)phenoxy)furan-2-carboxamide | 160 |

## Claims

1. A compound of any of formulae (I) to (V): 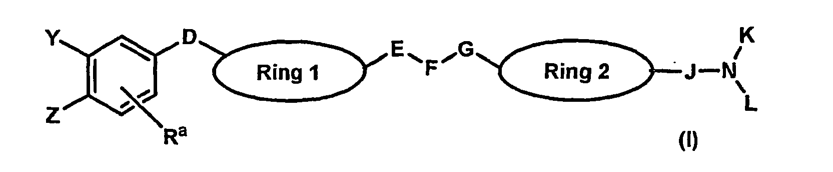 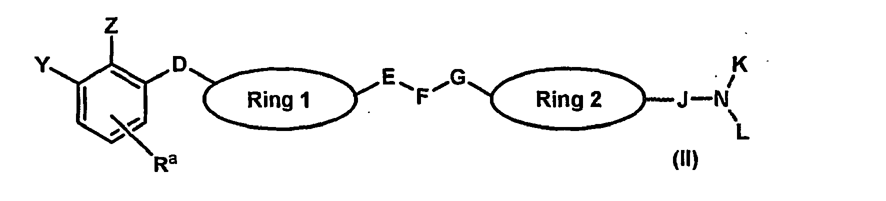 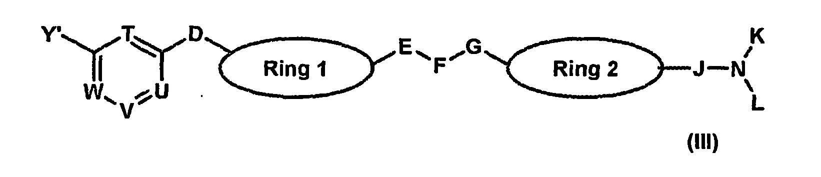 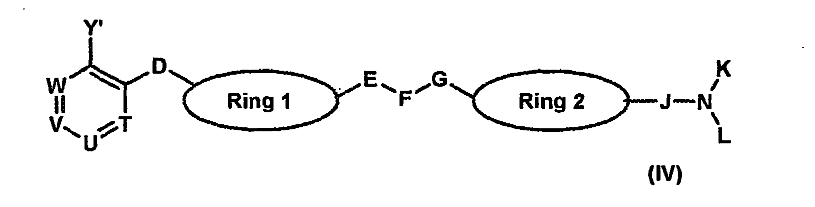 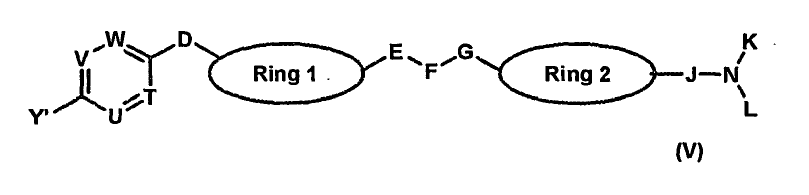 wherein
D is -(CH₂)ₙ-, -C(=X)-, -O-, -S(O)ₘ-, -C(=X)N(R^{e})-; -C(R^{b})₂-_{,} -C(R^{b})=C(R^{b})-, or -CH(R^{b})CH(R^{b})-;
E is optionally present and is -(CH₂)ₙ-, -N(R^{d})-, -(CH₂)ₙN(R^{d})- or -N(R^{d})(CH₂)ₙ-;
F is -C(=X)- or -N(R^{d})-;
G is -(CH₂)ₙ-, -N(R^{d})-, -(CH₂)ₙN(R^{d})- or -N(R^{d})(CH₂)ₙ;
J is a bond, a bridged heterocycloalkyl or a group of formula (i) or formula (ii): 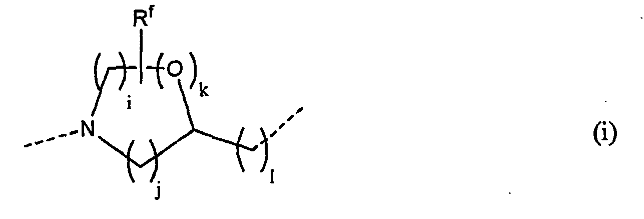 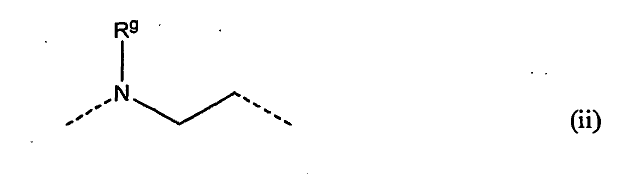 wherein
R^{f} is optionally present and is oxo (=O);
R^{g} is alkyl or -C(O)-alkyl;
i and j are the same or different and are each 1 or 2; and
k and I are the same or different and are each 0 or 1;
K and L are the same or different and are each hydrogen, alkyl, cycloalkyl, -alkyl-cycloalkyl or heterocycloalkyl optionally substituted with R^{a};
or K and L taken together form heterocycloalkyl;
each R^{a} is the same or different and is hydrogen, halogen, alkyl, aryl, hydroxy, alkoxy, -alkoxy-(CH₂)ₙC(=O)OR^{b}, -O-aryl, -C(=X)R^{c}, -NO₂, -CN, -N(R^{c})C(=X)R^{c}, -C(=X)N(R^{c})₂, -S(O)₂N(R^{c})₂ or -N(R^{e})₂;
each R^{b} is the same or different and is hydrogen or alkyl;
each R^{c} is the same or different and is alkyl, cycloalkyl, -alkyl-aryl, -alkyl-cycloalkyl or aryl optionally substituted with R^{a};
each R^{d} is the same or different and is hydrogen, alkyl or aryl optionally substituted with R^{a};
each R^{e} is the same or different and is hydrogen, alkyl; or R^{e} is aryl or heteroaryl, either of which is optionally substituted with R^{a};
one or two of T, U, V and W is nitrogen, and the others are each C(R^{a});
each X is the same or different and is oxygen or sulphur;
Y and Z are the same or different and are each hydrogen, halogen, alkyl, hydroxy, alkoxy, -CN, -N(R^{d})C(=X)R^{c}, -C(=X)N(R^{c})(R^{d}), -S(O)ₘ-R^{c}, -N(R^{c})(R^{d})S(O)₂, -S(O)₂N(R^{c})(R^{d}), -N(R^{e})₂, -Si(R^{c})₃, -alkyl-Si(R^{c})₃, aryl optionally substituted with R^{a} or -O-aryl optionally substituted with R^{a};
Y' is -Si(R^{c})₃ or -alkyl-Si(R^{c})₃;
Rings 1 and 2 are the same or different and are each arylene or heteroarylene, either of which is optionally substituted with R^{a};
each m is the same or different and is 0, 1 or 2; and
each n is the same or different and is 0, 1, 2 or 3;
with the provisos that: at least one of Y and Z comprises a silicon atom; the compound does not contain a N-N single bond; and the compound is not 5-[2-bromo-5-(trimethylsilyl)phenoxy]-*N*-{2-[(2-aminoethyl)propylamino)]-4,6-dimethoxy-1,3-pyrimidin-5-yl}furan-2-carboxamide; or a pharmaceutically acceptable salt thereof.

2. A compound according to claim 1, wherein D is -O-, -S- or -CH₂-.

3. A compound according to claim 1 or claim 2, wherein E is absent.

4. A compound according to any preceding claim, wherein F is -C(O)-.

5. A compound according to any preceding claim, wherein G is -N(R^{d})-.

6. A compound according to claim 5, wherein R^{d} is hydrogen.

7. A compound according to any preceding claim, wherein J is a group of formula (i) and i, j and k are, respectively: 2, 2 and 0; 1, 1 and 0; 2, 1 and 0; or 2, 1 and 1.

8. A compound according to claim 7, wherein i, j and k are 2, 2 and 0 respectively, and R^{f} is present.

9. A compound according to any of claims 1 to 6, wherein J is a group of formula (ii) and R^{g} is ethyl, propyl, ethanoyl or propanoyl.

10. A compound according to any of claims 1 to 6, wherein J is azabicyclo[3.2.1]octan-3-yl.

11. A compound according to any preceding claim, wherein K is methyl and L is hydrogen or methyl; or K-N-L taken together form azetidinyl.

12. A compound according to any preceding claim, wherein the compound is of any of formulae (I) to (III), and wherein one or two of T, U, V and W is nitrogen, and the others are each CH.

13. A compound according to any preceding claim, wherein Ring 1 is furanylene.

14. A compound according to any preceding claim, wherein Ring 2 is phenylene, pyrimidylene or pyridinylene, any of which is optionally substituted.

15. A compound according to claim 14, wherein Ring 2 is substituted 1, 2 or 3 times, the substituents being the same or different and selected from alkoxy and halogen.

16. A compound according to claim 1, selected from:
N-(2-(4-aminopiperidin-1-yl)-4,6-dimethoxypyrimidin-5-yl)-5-(2-methyl-5-(trimethylsilyl)phenoxy)furan-2-carboxamide;
N-(4,6-dimethoxy-2-(4-(methylamino)piperidin-1-yl)pyrimidin-5-yl)-5-(2-methyl-5-(trimethylsilyl)phenoxy)furan-2-carboxamide;
N-(2-(4-(dimethylamino)piperidin-1-yl)-4,6-dimethoxypyrimidin-5-yl)-5-(2-methyl-5-(trimethylsilyl)phenoxy)furan-2-carboxamide;
N-(2-(4-(azetidin-1-yl)piperidin-1-yl)-4,6-dimethoxypyrimidin-5-yl)-5-(2-methyl-5-(trimethylsilyl)phenoxy)furan-2-carboxamide;
N-(2-(4-(dimethylamino)piperidin-1-yl)-4,6-dimethoxypyrimidin-5-yl)-5-(3-(trimethylsilyl)phenoxy)furan-2-carboxamide;
N-(2-(4-(dimethylamino)piperidin-1-yl)-4,6-dimethoxypyrimidin-5-yl)-5-(3-(ethyldimethylsilyl)phenoxy)furan-2-carboxamide;
N-(2-(3-aminoazetidin-1-yl)-4,6-dimethoxypyrimidin-5-yl)-5-(2-methyl-5-(trimethylsilyl)phenoxy)furan-2-carboxamide;
N-(4,6-dimethoxy-2-(3-(methylamino)azetidin-1-yl)pyrimidin-5-yl)-5-(2-methyl-5-(trimethylsilyl)phenoxy)furan-2-carboxamide;
N-(2-(3-(dimethylamino)azetidin-1-yl)-4,6-dimethoxypyrimidin-5-yl)-5-(2-methyl-5-(trimethylsilyl)phenoxy)furan-2-carboxamide;
N-(2-(3-(azetidin-1-yl)azetidin-1-yl)-4,6-dimethoxypyrimidin-5-yl)-5-(2-methyl-5-(trimethylsilyl)phenoxy)furan-2-carboxamide;
N-(2-(3-(azetidin-1-yl)azetidin-1-yl)-4,6-dimethoxypyrimidin-5-yl)-5-(3-(trimethylsilyl)phenoxy)furan-2-carboxamide;
N-(2-(3-(azetidin-1-yl)azetidin-1-yl)-4,6-dimethoxypyrimidin-5-yl)-5-(3-(ethyldimethylsilyl)phenoxy)furan-2-carboxamide;
N-(2-(3-aminopyrrolidin-1-yl)-4,6-dimethoxypyrimidin-5-yl)-5-(2-methyl-5-(trimethylsilyl)phenoxy)furan-2-carboxamide;
N-(4,6-dimethoxy-2-(3-(methylamino)pyrrolidin-1-yl)pyrimidin-5-yl)-5-(2-methyl-5-(trimethylsilyl)phenoxy)furan-2-carboxamide;
N-(2-(3-(dimethylamino)pyrrolidin-1-yl)-4,6-dimethoxypyrimidin-5-yl)-5-(2-methyl-5-(trimethylsilyl)phenoxy)furan-2-carboxamide;
N-(2-(3-(azetidin-1-yl)pyrrolidin-1-yl)-4,6-dimethoxypyrimidin-5-yl)-5-(2-methyl-5-(trimethylsilyl)phenoxy)furan-2-carboxamide;
N-(2-(3-(dimethylamino)pyrrolidin-1-yl)-4,6-dimethoxypyrimidin-5-yl)-5-(3-(trimethylsilyl)phenoxy)furan-2-carboxamide;
N-(2-(3-(dimethylamino)pyrrolidin-1-yl)-4,6-dimethoxypyrimidin-5-yl)-5-(3-(ethyldimethylsilyl)phenoxy)furan-2-carboxamide;
N-(2-(3-(aminomethyl)pyrrolidin-1-yl)-4,6-dimethoxypyrimidin-5-yl)-5-(2-methyl-5-(trimethylsilyl)phenoxy)furan-2-carboxamide;
N-(4,6-dimethoxy-2-(3-((methylamino)methyl)pyrrolidin-1-yl)pyrimidin-5-yl)-5-(2-methyl-5-(trimethylsilyl)phenoxy)furan-2-carboxamide;
N-(2-(3-((dimethylamino)methyl)pyrrolidin-1-yl)-4,6-dimethoxypyrimidin-5-yl)-5-(2-methyl-5-(trimethylsilyl)phenoxy)furan-2-carboxamide;
N-(2-(3-(azetidin-1-ylmethyl)pyrrolidin-1-yl)-4,6-dimethoxypyrimidin-5-yl)-5-(2-methyl-5-(trimethylsilyl)phenoxy)furan-2-carboxamide;
N-(2-(3-((dimethylamino)methyl)pyrrolidin-1-yl)-4,6-dimethoxypyrimidin-5-yl)-5-(3-(trimethylsilyl)phenoxy)furan-2-carboxamide;
N-(2-(3-((dimethylamino)methyl)pyrrolidin-1-yl)-4,6-dimethoxypyrimidin-5-yl)-5-(3-(ethyldimethylsilyl)phenoxy)furan-2-carboxamide;
N-(2-(2-(aminomethyl)morpholin-4-yl)-4,6-dimethoxypyrimidin-5-yl)-5-(2-methyl-5-(trimethylsilyl)phenoxy)furan-2-carboxamide;
N-(4,6-dimethoxy-2-(2-((methylamino)methyl)morpholin-4-yl)pyrimidin-5-yl)-5-(2-methyl-5-(trimethylsilyl)phenoxy)furan-2-carboxamide;
N-(2-(2-((dimethylamino)methyl)morpholin-4-yl)-4,6-dimethoxypyrimidin-5-yl)-5-(2-methyl-5-(trimethylsilyl)phenoxy)furan-2-carboxamide;
N-(2-(2-(azetidin-1-ylmethyl)morpholin-4-yl)-4,6-dimethoxypyrimidin-5-yl)-5-(2-methyl-5-(trimethylsilyl)phenoxy)furan-2-carboxamide;
N-(2-(2-((dimethylamino)methyl)morpholin-4-yl)-4,6-dimethoxypyrimidin-5-yl)-5-(3-(trimethylsilyl)phenoxy)furan-2-carboxamide;
N-(2-(2-((dimethylamino)methyl)morpholin-4-yl)-4,6-dimethoxypyrimidin-5-yl)-5-(3-(ethyldimethylsilyl)phenoxy)furan-2-carboxamide;
N-(2-(4-(dimethylamino)-2-oxopiperidin-1-yl)-4,6-dimethoxypyrimidin-5-yl)-5-(2-methyl-5-(trimethylsilyl)phenoxy)furan-2-carboxamide;
N-(4,6-dimethoxy-2-(4-(methylamino)-2-oxopiperidin-1-yl)pyrimidin-5-yl)-5-(2-methyl-5-(trimethylsilyl)phenoxy)furan-2-carboxamide;
N-(2-(4-(dimethylamino)-2-oxopiperidin-1-yl)-4,6-dimethoxypyrimidin-5-yl)-5-(3-(trimethylsilyl)phenoxy)furan-2-carboxamide;
N-(4,6-dimethoxy-2-(4-(methylamino)-2-oxopiperidin-1-yl)pyrimidin-5-yl)-5-(3-(trimethylsilyl)phenoxy)furan-2-carboxamide;
N-(2-(4-(dimethylamino)-2-oxopiperidin-1-yl)-4,6-dimethoxypyrimidin-5-yl)-5-(3-(ethyldimethylsilyl)phenoxy)furan-2-carboxamide;
N-(2-((2-aminoethyl)(propyl)amino)-4,6-dimethoxypyrimidin-5-yl)-5-(2-methyl-5-(trimethylsilyl)phenoxy)furan-2-carboxamide;
N-(4,6-dimethoxy-2-((2-(methylamino)ethyl)(propyl)amino)pyrimidin-5-yl)-5-(2-methyl-5-(trimethylsilyl)phenoxy)furan-2-carboxamide;
N-(2-((2-(dimethylamino)ethyl)(propyl)amino)-4,6-dimethoxypyrimidin-5-yl)-5-(2-methyl-5-(trimethylsilyl)phenoxy)furan-2-carboxamide;
N-(2-((2-(azetidin-1-yl)ethyl)(propyl)amino)-4,6-dimethoxypyrimidin-5-yl)-5-(2-methyl-5-(trimethylsilyl)phenoxy)furan-2-carboxamide;
N-(2-((2-(dimethylamino)ethyl)(propyl)amino)-4,6-dimethoxypyrimidin-5-yl)-5-(3-(trimethylsilyl)phenoxy)furan-2-carboxamide;
N-(2-((2-(dimethylamino)ethyl)(propyl)amino)-4,6-dimethoxypyrimidin-5-yl)-5-(3-(ethyldimethylsilyl)phenoxy)furan-2-carboxamide;
N-(2-((2-aminoethyl)(ethyl)amino)-4,6-dimethoxypyrimidin-5-yl)-5-(2-methyl-5-(trimethylsilyl)phenoxy)furan-2-carboxamide;
N-(2-(ethyl(2-(methylamino)ethyl)amino)-4,6-dimethoxypyrimidin-5-yl)-5-(2-methyl-5-(trimethylsilyl)phenoxy)furan-2-carboxamide;
N-(2-((2-(dimethylamino)ethyl)(ethyl)amino)-4,6-dimethoxypyrimidin-5-yl)-5-(2-methyl-5-(trimethylsilyl)phenoxy)furan-2-carboxamide;
N-(2-((2-(azetidin-1-yl)ethyl)(ethyl)amino)-4,6-dimethoxypyrimidin-5-yl)-5-(2-methyl-5-(trimethylsilyl)phenoxy)furan-2-carboxamide;
N-(2-((2-(dimethylamino)ethyl)(ethyl)amino)-4,6-dimethoxypyrimidin-5-yl)-5-(3-(trimethylsilyl)phenoxy)furan-2-carboxamide;
N-(2-((2-(dimethylamino)ethyl)(ethyl)amino)-4,6-dimethoxypyrimidin-5-yl)-5-(3-(ethyldimethylsilyl)phenoxy)furan-2-carboxamide;
N-(2-(N-(2-(dimethylamino)ethyl)propionamido)-4,6-dimethoxypyrimidin-5-yl)-5-(2-methyl-5-(trimethylsilyl)phenoxy)furan-2-carboxamide;
N-(2-(N-(2-(dimethylamino)ethyl)acetamido)-4,6-dimethoxypyrimidin-5-yl)-5-(2-methyl-5-(trimethylsilyl)phenoxy)furan-2-carboxamide;
N-(2-(N-(2-(dimethylamino)ethyl)propionamido)-4,6-dimethoxypyrimidin-5-yl)-5-(3-(trimethylsilyl)phenoxy)furan-2-carboxamide;
N-(2-(N-(2-(dimethylamino)ethyl)acetamido)-4,6-dimethoxypyrimidin-5-yl)-5-(3-(trimethylsilyl)phenoxy)furan-2-carboxamide;
N-(2-(N-(2-aminoethyl)propionamido)-4,6-dimethoxypyrimidin-5-yl)-5-(2-methyl-5-(trimethylsilyl)phenoxy)furan-2-carboxamide;
N-(2-(N-(2-aminoethyl)acetamido)-4,6-dimethoxypyrimidin-5-yl)-5-(2-methyl-5-(trimethylsilyl)phenoxy)furan-2-carboxamide;
5-(2-methyl-5-(trimethylsilyl)phenoxy)-N-(2-(3-(dimethylamino)-8-aza-bicyclo[3.2.1]octan-8-yl)-4,6-dimethoxypyrimidin-5-yl)furan-2-carboxamide;
N-(2-(ethyl(2-(isopropylamino)ethyl)amino)-4,6-dimethoxypyrimidin-5-yl)-5-(2-methyl-5-(trimethylsilyl)phenoxy)furan-2-carboxamide;
N-(2-(3-(isopropylamino)pyrrolidin-1-yl)-4,6-dimethoxypyrimidin-5-yl)-5-(2-methyl-5-(trimethylsilyl)phenoxy)furan-2-carboxamide;
*N*-(4,6-Dimethoxy-2-(methyl(1-methylpyrrolidin-3-yl)amino)pyrimidin-5-yl)-5-(2-methyl-5-(trimethylsilyl)phenoxy)furan-2-carboxamide; and
N-(4,6-dimethoxy-2-(imidazolidin-2-on-1-yl)pyrimidin-5-yl)-5-(2-methyl-5-(trimethylsilyl)phenoxy)furan-2-carboxamide;

17. A compound according to any preceding claim, which is in the form of a single enantiomer, diastereomer or tautomer.

18. A compound according to any preceding claim, for therapeutic use.

19. A pharmaceutical composition comprising a compound of any of claims 1 to 18 and a pharmaceutically acceptable diluent or carrier.

20. Use of a compound of any of claims 1 to 17, for the manufacture of a medicament for cancer therapy.

21. Use of a compound of any of claims 1 to 17, for the manufacture of a medicament for the treatment or prevention of endometriosis, uterine myoma, an ovarian disease, a mammary cystic disease, prostatic hypertrophy, amenorrhoea, precocious puberty, premenstrual syndrome, a sex-steroid-dependent pathophysiology or benign prostatic hyperplasia, or to arrest spermatogenesis.

22. Use according to claim 21, for the treatment or prevention of endometriosis with pain, polycystic ovarian disease or secondary amenorrhoea.

23. Use of a compound of any of claims 1 to 17, for the manufacture of a medicament for the treatment or prevention of Alzheimer's disease.

24. Use of a compound of any of claims 1 to 17, for the manufacture of a medicament for the treatment or prevention of HIV infection or AIDS.

25. Use of a compound of any of claims 1 to 17, for the manufacture of a medicament for the treatment or prevention of a disease caused by thymic malfunction.

26. Use according to claim 25, for the treatment or prevention of multiple sclerosis, rheumatoid arthritis or type 1 diabetes.

## Patentansprüche

1. Verbindung einer der Formeln (I) bis (V) 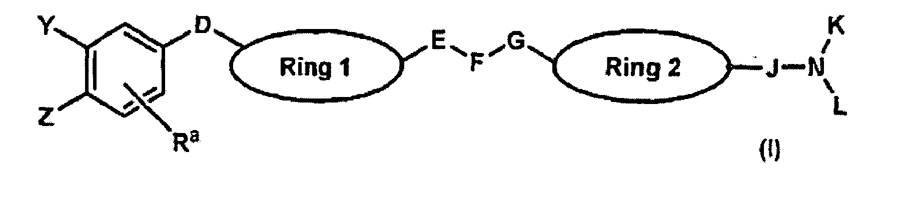 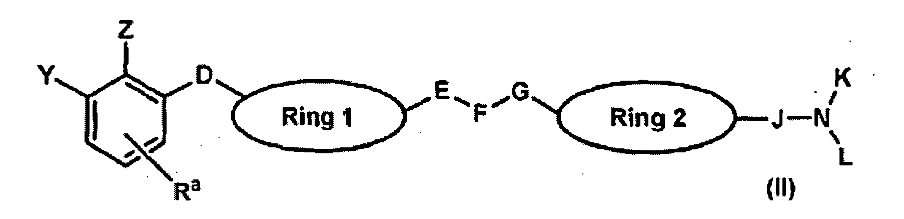 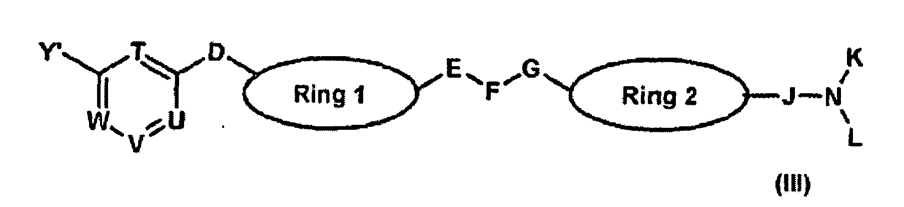 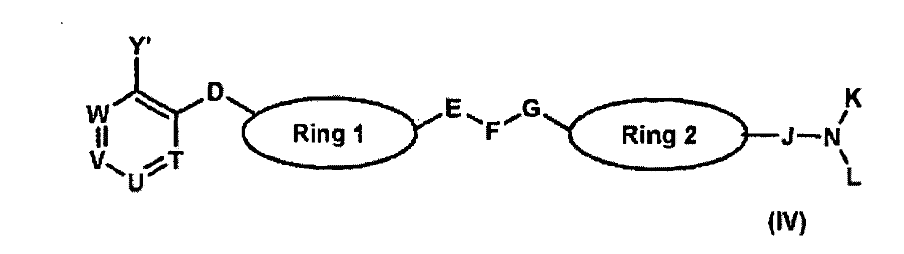 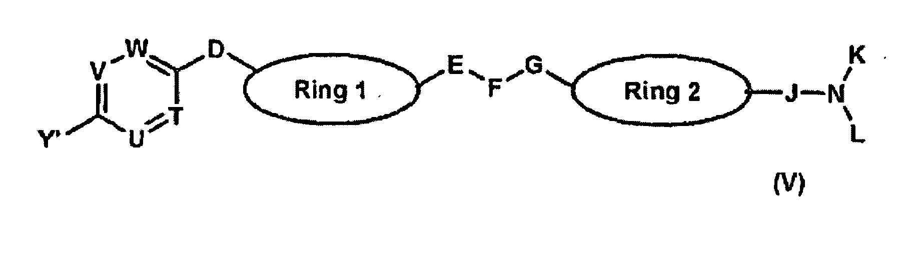 wobei D -(CH₂)ₙ-, -C(=X)-, -O-, -S(O)ₘ-, -C(=X)N(R^{e})-, -C(R^{b})₂-, -C(R^{b})=C(R^{b})- oder -CH(R^{b})CH(R^{b})- ist;
E wahlweise anwesend ist und -(CH₂)ₙ-, -N(R^{d})-, -(CH₂)ₙN(R^{d})- oder -N(R^{d})(CH₂)ₙ- ist;
F -C(=X)- oder -N(R^{d})- ist;
G -(CH₂)ₙ-, -N(R^{d})-, -(CH₂)ₙN(R^{d})- oder -N(R^{d})(CH₂)ₙ ist,
J eine Bindung, ein überbrücktes Heterocycloalkyl oder eine Gruppe der Formel (i) oder der Formel (ii) ist: 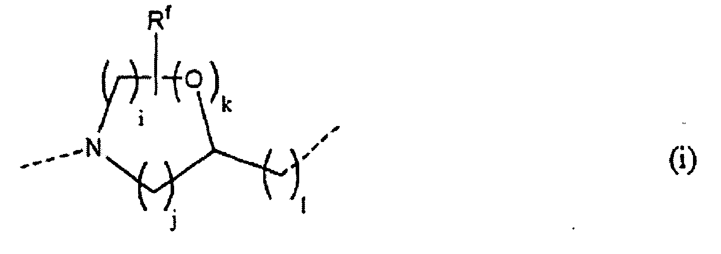 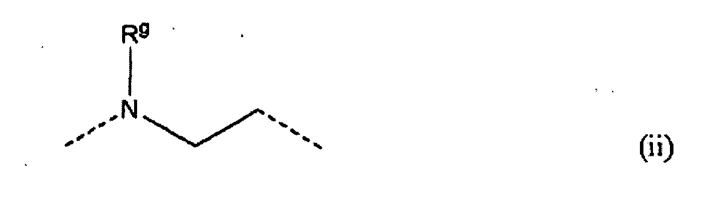 wobei
R^{f} wahlweise anwesend ist und eine Oxogruppe ist (=O);
R^{g} Alkyl oder -C(O)-Alkyl ist;
i und j gleich oder verschieden sind und jeweils 1 oder 2 sind; und
k und I gleich oder verschieden sind und jeweils 0 oder 1 sind;
K und L gleich oder verschieden sind und jeweils Wasserstoff, Alkyl, Cycloalkyl, -Alkyl-cycloalkyl oder Heterocycloalkyl wahlweise mit R^{a} substituiert sind; oder K und L zusammen ein Heterocycloalkyl bilden;
jedes R^{a} gleich oder verschieden ist und Wasserstoff, Halogen, Alkyl, Aryl, Hydroxy, Alkoxy, -Alkoxy-(CH₂)ₙC(=O)OR^{b}, -O-Aryl, -C(=X)R^{c}, -NO₂, -CN, -N(R^{c})C(=X)R^{c}, -C(=X)N(R^{c})₂, -S(O)₂N(R^{c})₂, oder -N(R^{e})₂ ist;
jedes R^{b} gleich oder verschieden ist und Wasserstoff oder Alkyl ist;
jedes R^{c} gleich oder verschieden ist und Alkyl, Cycloalkyl, -Alkyl-aryl, -Alkyl-cycloalkyl oder Aryl wahlweise mit R^{a} substituiert ist;
jedes R^{d} gleich oder verschieden ist und Wasserstoff, Alkyl oder Aryl wahlweise mit R^{a} substituiert ist;
jedes R^{e} gleich oder verschieden ist und Wasserstoff, Alkyl ist; oder R^{e} Aryl oder Heteroaryl ist, von denen jedes wahlweise mit R^{a} substituiert ist;
eines oder zwei aus T, U, V und W Stickstoff ist und die anderen jeweils C(R^{a}) sind;
jedes X gleich oder verschieden ist und Sauerstoff oder Schwefel ist;
Y und Z gleich oder verschieden sind und jeweils Wasserstoff, Halogen, Alkyl, Hydroxy, Alkoxy, -CN, -N(R^{d})C(=X)R^{c}, -C(=X)N(R^{c})(R^{d}), -S(O)ₘ-R^{c}, -N(R^{c})(R^{d})S(O)₂, -S(O)₂N(R^{c})(R^{d}), -N(R^{e})₂, -Si(R^{c})₃, -Alkyl-Si(R^{c})₃, Aryl wahlweise mit R^{a} substituiert oder -O-Aryl wahlweise mit R^{a} substituiert ist;
Y' -Si(R^{c})₃ oder -Alkyl-Si(R^{c})₃ ist;
die Ringe 1 und 2 gleich oder verschieden sind und jeweils Arylen oder Heteroarylen sind, von denen jeder wahlweise mit R^{a} substituiert ist;
jedes m gleich oder verschieden ist und 0, 1 oder 2 ist; und
jedes n gleich oder verschieden ist und 0, 1, 2 oder 3 ist;
mit den Maßgaben, dass mindestens eines von Y und Z ein Siliziumatom aufweist; die Verbindung keine N-N Einfachbindung enthält;
die Verbindung kein 5-[2-Brom-5-(trimethylsilyl)phenoxy]-*N*-{2-[(2-aminoethyl)-propylamino)]-4,6-dimethoxy-1,3-pyrimidin-5-yl}furan-2-carboxamid, oder ein pharmazeutisch verträgliches Salz davon ist.

2. Verbindung gemäß Anspruch 1, wobei D -O-, -S- oder -CH₂- ist.

3. Verbindung gemäß Anspruch 1 oder 2, wobei E abwesend ist.

4. Verbindung gemäß einem vorhergehenden Anspruch, wobei F -C(O)- ist.

5. Verbindung gemäß einem vorhergehenden Anspruch, wobei G -N(R^{d})- ist.

6. Verbindung gemäß Anspruch 5, wobei R^{d} Wasserstoff ist.

7. Verbindung gemäß einem vorhergehenden Anspruch, wobei J eine Gruppe der Formel (i) ist und i, j und k jeweils: 2, 2 bzw. 0; 1, 1 bzw. 0; 2, 1 bzw. 0; oder 2, 1 bzw. 1 sind.

8. Verbindung gemäß Anspruch 7, wobei i, j und k jeweils 2, 2 bzw. 0 sind und R^{f} anwesend ist.

9. Verbindung gemäß einem der Ansprüche 1 bis 6, wobei J eine Gruppe der Formel (ii) ist und R^{g} Ethyl, Propyl, Ethanoyl oder Propanoyl ist.

10. Verbindung gemäß einem der Ansprüche 1 bis 6, wobei J Azabicyclo[3.2.1]octan-3-yl ist.

11. Verbindung gemäß einem vorhergehenden Anspruch, wobei K Methyl und L Wasserstoff oder Methyl ist; oder K-N-L zusammen Azetidinyl bilden.

12. Verbindung gemäß einem vorhergehenden Anspruch, wobei die Verbindung eine der Formeln (I) bis (III) aufweist und wobei eine oder zwei aus T, U, V und W Stickstoff ist und die anderen jeweils CH sind.

13. Verbindung gemäß einem vorhergehenden Anspruch, wobei Ring 1 Furanylen ist.

14. Verbindung gemäß einem vorhergehenden Anspruch, wobei Ring 2 Phenylen, Pyrimidylen oder Pyridinylen ist, von denen irgendeines bzw. jedes wahlweise substituiert ist.

15. Verbindung gemäß Anspruch 14, wobei Ring 2 1- 2- oder 3-mal substituiert ist, die Substituenten gleich oder verschieden sind und aus Alkoxy und Halogen ausgewählt sind.

16. Verbindung gemäß Anspruch 1, ausgewählt aus:
N-(2-(4-Aminopiperidin-1-yl)-4,6-dimethoxypyrimidin-5-yl)-5-(2methyl-5-(trimethylsilyl)phenoxy)furan-2-carboxamid;
N-(4,6-Dimethoxy-2-(4-(methylamino)piperidin-1-y)pyrimidin-5-yl)-5-(2-methyl-5-(trimethylsilyl)phenoxy)furan-2-carboxamid;
N-(2-(4-(Dimethylamino)piperidin-1-yl)-4,6-dimethoxypyrimidin-5-yl)-5-(2-methyl-5-(trimethylsilyl)phenoxy)furan-2-carboxamid;
N-(2-(4-Azetidin-1-yl)piperidin-1-yl)-4,6-dimethoxypyrimidin-5-yl)-5-(2-methyl-5-(trimethylsilyl)phenoxy)furan-2-carboxamid;
N-(2-(4-Dimethylamino)piperidin-1-yl)-4,6-dimethoxypyrimidin-5-yl)-5-(3-trimethylsilyl)phenoxy)furan-2-carboxamid;
N-(2-(4-Dimethylamino)piperidin-1-yl)-4,6-dimethoxypyrimidin-5-yl)-5-(3-(ethyldimethylsilyl)phenoxy)furan-2-carboxamid;
N-(2-(3-Aminoazetidin-1-yl)-4,6-dimethoxypyrimidin-5-yl)-5-(2-methyl-5-(trimethylsilyl)phenoxy)furan-2-carboxamid;
N-(4,6-Dimethoxy-2-(3-methylamino)azetidin-1-yl)pyrimidin-5-yl)-5-(2-methyl-5-(trimethylsilyl)phenoxy)furan-2-carboxamid;
N-(2-(3-Dimethylamino)azetidin-1-yl)-4,6-dimethoxypyrimidin-5-yl)-5-(2-methyl-5-(trimethylsilyl)phenoxy)furan-2-carboxamid;
N-(2-(3-(Azetidin-1-yl)azetidin-1-yl)-4,6-dimethoxypyrimidin-5-yl)-5-(2-methyl-5-(trimethylsilyl)phenoxy)furan-2-carboxamid;
N-(2-(3-Azetidin-1-yl)azetidin-1-yl)-4,6-dimethoxypyrimidin-5-yl)-5-(3-(trimethylsilyl)phenoxy)furan-2-carboxamid;
N-(2-(3-Azetidin-1-yl)azetidin-1-yl)-4,6-dimethoxypyrimidin-5-yl)-5-(3-(ethyldimethylsilyl)phenoxy)furan-2-carboxamid;
N-(2-(3-Aminopyrrolidin-1-yl)-4,6-dimethoxypyrimidin-5-yl)-5-(2-methyl-5-(trimethylsilyl)phenoxy)furan-2-carboxamid;
N-(4,6-Dimethoxy-2-(3-(Methylamino)pyrrolidin-1-yl)pyrimidin-5-yl)-5-(2-methyl-5-(trimethylsilyl)phenoxy)furan-2-carboxamid;
N-(2-(3-Dimethylamino)pyrrolidin-1-yl)-4,6-dimethoxypyrimidin-5-yl)-5-(2-methyl-5-(trimethylsilyl)phenoxy)furan-2-carboxamid;
N-(2-(3-Azetidin-1-yl)pyrrolidin-1-yl)-4,6-dimethoxypyrimidin-5-yl)-5-(2-methyl-5-(trimethylsilyl)phenoxy)furan-2-carboxamid;
N-(2-(3-Dimethylamino)pyrrolidin-1-yl)-4,6-dimethoxypyrimidin-5-yl)-5-(3-(trimethylsilyl)phenoxy)furan-2-carboxamid;
N-(2-(3-Dimethylamino)pyrrolidin-1-y)-4,6-dimethoxypyrimidin-5-yl)-5-(3-(ethyldimethylsilyl)phenoxy)furan-2-carboxamid;
N-(2-(3-(Aminomethyl)pyrrolidin-1-yl)-4,6-dimethoxypyrimidin-5-yl)-5-(2-methyl-5-(trimethylsilyl)phenoxy)furan-2-carboxamid;
N-(4,6-Dimethoxy-2-(3-((methylamino)methyl)pyrrolidin-1-yl)pyrimidin-5-yl)-5-(2-methyl-5-(trimethylsilyl)phenoxy)furan-2-carboxamid;
N-(2-(3-((Dimethylamino)methyl)pyrrolidin-1-yl)-4,6-dimethoxypyrimidin-5-yl)-5-(2-methyl-5-(trimethylsilyl)phenoxy)furan-2-carboxamid;
N-(2-(3-Azetidin-1-ylmethyl)pyrrolidin-1-yl)-4,6-dimethoxypyrimidin-5-yl)-5-(2-methyl-5-(trimethylsilyl)phenoxy)furan-2-carboxamid;
N-(2-(3-((Dimethylamino)methyl)pyrrolidin-1-yl)-4,6-dimethoxyprimidin-5-yl)-5-(3-(trimethylsilyl)phenoxy)furan-2-carboxamid;
N-(2-(3-((Dimethylamino)methyl)pyrrolidin-1-yl)-4,6-dimethoxypyrimidin-5-yl)-5-(3-(ethyldimethylsilyl)phenoxy)furan-2-carboxamid;
N-(2-(2-(Aminomethyl)morpholin-4-yl)-4,6-dimethoxypyrimidin-5-yl)-5-(2-methyl-5-(trimethylsilyl)phenoxy)furan-2-carboxamid;
N-(4,6-Dimethoxy-2-(2-((methylamino)methyl)morpholin-4-yl)pyrimidin-5-yl)-5-(2-methyl-5-(trimethylsilyl)phenoxy)furan-2-carboxamid;
N-(2(2-((Dimethylamino)methyl)morpholin-4-yl)-4,6-dimethoxypyrimidin-5-yl)-5-(2-methyl-5-(trimethylsilyl)phenoxy)furan-2-carboxamid;
N-(2-(2-(Azetidin-1-ylmethyl)morpholin-4-yl)-4,6-dimethoxypyrimidin-5-yl)-5-(2-methyl-5-(trimethylsilyl)phenoxy)furan-2-carboxamid;
N-(2-(2-((Dimethylamino)methyl)morpholin-4-yl)-4,6-dimethoxypyrimidin-5-yl)-5-(3-(trimethylsilyl)phenoxy)furan-2-carboxamid;
N-(2-(2-((Dimethylamino)methyl)morpholin-4-yl)-4,6-dimethoxypyrimidin-5-yl)-5-(3-(ethyldimethylsilyl)phenoxy)furan-2-carboxamid;
N-(2-(4-(Dimethylamino)-2-oxopiperidin-1-yl)-4,6-dimethoxypyrimidin-5-yl)-5-(2-methyl-5-(trimethylsilyl)phenoxy)furan-2-carboxamid;
N-(4,6-Dimethoxy-2-(4-(methylamino)-2-oxopiperidin-1-yl)pyrimidin-5-yl)-5-(2-methyl-5-(trimethylsilyl)phenoxy)furan-2-carboxamid;
N-(2-(4-Dimethylamino)-2-oxopiperidin-1-yl)-4,6-dimethoxypyrimdin-5-yl)-5-(3-(trimethylsilyl)phenoxy)furan-2-carboxamid;
N-(4,6-Dimethoxy-2-(4-methylamino)-2-oxopiperidin-1-yl)pyrimidin-5-yl)-5-(3-(trimethylsilyl)phenoxy)furan-2-carboxamid;
N-(2-(4-(Dimethylamino)-2-oxopiperidin-1-yl)-4,6-dimethoxypyrimidin-5-yl)-5-(3-(ethyldimethylsilyl)phenoxy)furan-2-carboxamid;
N-(2-((2-Aminoethyl)(propyl)amino)-4,6-dimethoxypyrimidin-5-yl)-5-(2-methyl-5-(trimethylsilyl)phenoxy)furan-2-carboxamid;
N-(4,6-Dimethoxy-2-((2-methylamino)ethyl)(propyl)amino)pyrimidin-5-yl)-5-(2-methyl-5-(trimethylsilyl)phenoxy)furan-2-carboxamid;
N-(2-((2-(Dimethylamino)ethyl)(propyl)amino)-4,6-dimethoxypyrimidin-5-yl)-5-(2-methyl-5-(trimethylsilyl)phenoxy)furan-2-carboxamid;
N-(2-((2-(Azetidin-1-yl)ethyl)(propyl)amino)-4,6-dimethoxypyrimidin-5-yl)-5-(2-methyl-5-(trimethylsilyl)phenoxy)furan-2-carboxamid;
N-(2-((2-(Dimethylamino)ethyl)(propyl)amino)-4,6-dimethoxypyrimidin-5-yl)-5-(3-(trimethylsilyl)phenoxy)furan-2-carboxamid;
N-(2-((2-Dimethylamino)ethyl)(propyl)amino)-4,6-dimethoxypyrimidin-5-yl)-5-(3-(ethyldimethylsilyl)phenoxy)furan-2-carboxamid;
N-(2-((2-Aminoethyl)(ethyl)amino)-4,6-dimethoxypyrimidin-5-yl)-5-(2-methyl-5-(trimethylsilyl)phenoxy)furan-2-carboxamid;
N-(2-(Ethyl(2-(methylamino)ethyl)amino)-4,6-dimethoxypyrimidin-5-yl)-5-(2-methyl-5-(trimethylsilyl)phenoxy)furan-2-carboxamid;
N-(2-((2-(Dimethylamino)ethyl)(ethyl)amino)-4,6-dimethoxypyrimidin-5-yl)-5-(2-methyl-5-(trimethylsilyl)phenoxy)furan-2-carboxamid;
N-(2-((2-(Azetidin-1-yl)ethyl)(ethyl)amino)-4,6-dimethoxypyrimidin-5-yl)-5-(2-methyl-5-(trimethylsilyl)phenoxy)furan-2-carboxamid;
N-(2-((2-(Dimethylamino)ethyl)(ethyl)amino-4,6-dimethoxypyrimidin-5-yl)-5-(3-(trimethylsilyl)phenoxy)furan-2-carboxamid;
N-(2-((2-(Dimethylamino)ethyl)(ethyl)amino)-4,6-dimethoxypyrimidin-5-yl)-5-(3-(ethyldimethylsilyl)phenoxy)furan-2-carboxamid;
N-(2-(N-(2-(Dimethylamino)ethyl)propionamido)-4,6-dimethoxypyrimidin-5-yl)-5-(2-methyl-5-(trimethylsilyl)phenoxy)furan-2-carboxamid;
N-(2-(N-(2-(Dimethylamino)ethyl)acetamido)-4,6-dimethoxypyrimidin-5-yl)-5-(2-methyl-5-(trimethylsilyl)phenoxy)furan-2-carboxamid;
N-(2-(N-(2-(Dimethylamino)ethyl)propionamido)-4,6-dimethoxypyrimidin-5-yl)-5-(3-(trimethylsilyl)phenoxy)furan-2-carboxamid;
N-(2-(N-(2-(Dimethylamino)ethyl)acetamido)-4,6-dimethoxypyrimidin-5-yl)-5-(3-(trimethylsilyl)phenoxy)furan-2-carboxamid;
N-(2-(N-(2-Aminoethyl)propionamido)-4,6-dimethoxypyrimidin-5-yl)-5-(2-methyl-5-(trimethylsilyl)phenoxy)furan-2-carboxamid;
N-(2-(N-(2-Aminoethyl)acetamido)-4,6-dimethoxypyrimidin-5-yl)-5-(2-methyl-5-(trimethylsilyl)phenoxy)furan-2-carboxamid;
5-(2-Methyl-5-(trimethylsilyl)phenoxy)-N-(2-(3-(dimethylamino)-8-azabicyclo[3.2.1]octan-8-yl)-4,6-dimethoxypyrimidin-5-yl)furan-2-carboxamid;
N-(2-(Ethyl(2-(isopropylamino)ethyl)amino)-4,6-dimethoxypyrimidin-5-yl)-5-(2-methyl-5-(trimethylsilyl)phenoxy)furan-2-carboxamid;
N-(2-(3-(Isopropylamino)pyrrolidin-1-yl)-4,6-dimethoxypyrimidin-5-yl)-5-(2-methyl-5-(trimethylsilyl)phenoxy)furan-2-carboxamid;
N-(4,6-Dimethoxy-2-(methyl(1-methylpyrrolidin-3-yl)amino)pyrimidin-5-yl)-5-(2-methyl-5-(trimethylsilyl)phenoxy)furan-2-carboxamid; und
N-(4,6-Dimethoxy-2-(imidazolidin-2-on-1-yl)pyrimidin-5-yl)-5-(2-methyl-5-(trimethylsilyl)phenoxy)furan-2-carboxamid.

17. Verbindung gemäß einem vorhergehenden Anspruch, die in Form eines einzelnen Enantiomers, Diastereomers oder Tautomers vorliegt.

18. Verbindung gemäß einem vorhergehenden Anspruch zur therapeutischen Verwendung.

19. Pharmazeutische Zusammensetzung, die eine Verbindung nach einem der Ansprüche 1 bis 18 und ein pharmazeutisch verträgliches Verdünnungsmittel oder einen pharmazeutisch verträglichen Träger umfasst.

20. Verwendung einer Verbindung nach einem der Ansprüche 1 bis 17 zur Herstellung eines Arzneimittels zur Krebstherapie.

21. Verwendung einer Verbindung nach einem der Ansprüche 1 bis 17 zur Herstellung eines Arzneimittels zur Behandlung oder Prävention von Endometriose, uterinem Myom, einer Ovarerkrankung, einer zystischen Brusterkrankung, prostatischer Hypertrophie, Amenorrhoe, vorzeitiger Pubertät, prämenstruellem Syndrom, einer Sexualsteroid-abhängigen Pathophysiologie oder gutartiger Prostatahyperplasie, oder um die Spermatogenese anzuhalten.

22. Verwendung gemäß Anspruch 21 zur Behandlung oder Prävention von Endometriose mit Schmerzen, polyzystischer Ovarerkrankung oder sekundärer Amenorrhoe.

23. Verwendung einer Verbindung nach einem der Ansprüche 1 bis 17 zur Herstellung eines Arzneimittels zur Behandlung oder Prävention von Alzheimer'scher Erkrankung.

24. Verwendung einer Verbindung nach einem der Ansprüche 1 bis 17 zur Herstellung eines Arzneimittels zur Behandlung oder Prävention von HIV-Infektion oder AIDS.

25. Verwendung einer Verbindung nach einem der Ansprüche 1 bis 17 zur Herstellung eines Arzneimittels zur Behandlung oder Prävention einer Erkrankung, die durch eine Thymus Fehlfunktion hervorgerufen wird.

26. Verwendung gemäß Anspruch 25 zur Behandlung oder Prävention von Multipler Sklerose, rheumatoider Arthritis oder Typ 1 Diabetes.

## Revendications

1. Composé répondant à l'une quelconque des formules (I) à (V) : 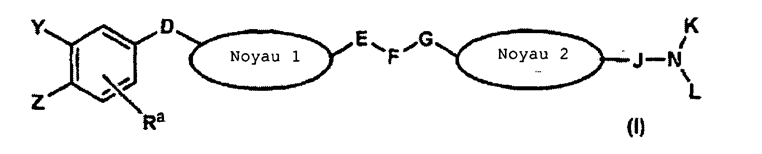 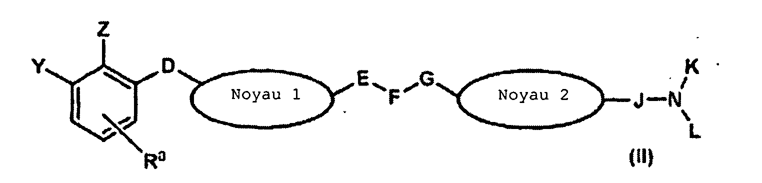 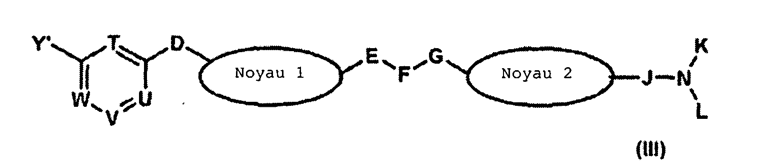 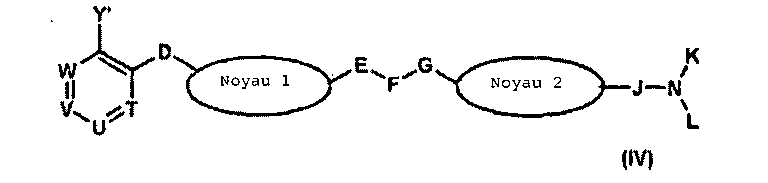 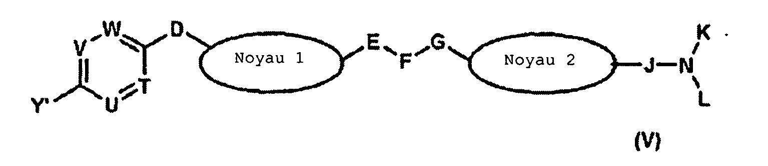 dans lesquelles
D représente un groupe -(CH₂)ₙ-, -C(=X)-, -O-, -S(O)ₘ-, -C(=X)N(R^{e})-, -C(R^{b})₂-, -C(R^{b})=C(R^{b})- ou -CH(R^{b})CH(R^{b})- ;
E est facultativement présent et représente un groupe -(CH₂)ₙ-, -N(R^{d})-, -(CH₂)ₙN(R^{d})- ou -N(R^{d})(CH₂)ₙ-;
F représente un groupe -C(=X)- ou -N(R^{d})- ;
G représente un groupe -(CH₂)ₙ-, -N(R^{d})-, -(CH₂)ₙN(R^{d})- ou -N(R^{d})(CH₂)ₙ ;
J représente une liaison, un groupe hétérocycloalkyle ponté ou un groupe de formule (i) ou de formule (ii) : 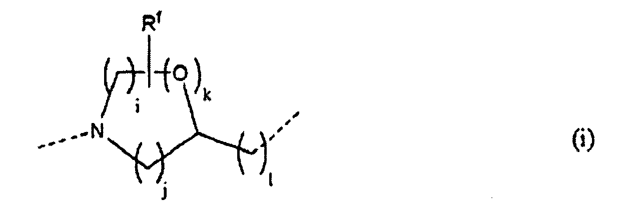 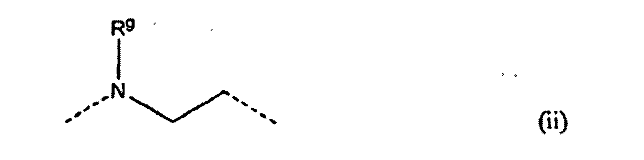 formules dans lesquelles
R^{f} est facultativement présent et représente un groupe oxo (=O) ;
R^{g} représente un groupe alkyle ou -C(O)-alkyle ;
i et j sont identiques ou différents et sont chacun égaux à 1 ou 2 ; et
k et l sont identiques ou différents et sont chacun égaux à 0 ou 1 ;
K et L sont identiques ou différents et représentent chacun un atome d'hydrogène, un groupe alkyle, cycloalkyle, -alkylcycloalkyle ou hétérocycloalkyle facultativement substitué avec R^{a} ;
ou bien K et L, pris conjointement, forment un groupe hétérocycloalkyle ;
les groupes R^{a} sont identiques ou différents et représentent chacun un atome d'hydrogène, un atome d'halogène, un groupe alkyle, aryle, hydroxy, alkoxy, -alkoxy-(CH₂)ₙC(=O)OR^{b}, -O-aryle, -C(=X)R^{c}, -NO₂, -CN, -N(R^{c})C(=X)R^{c}, -C(=X)N(R^{c})₂, -S(O)₂N(R^{c})₂ ou -N(R^{e})₂ ;
les groupes R^{b} sont identiques ou différents et représentent chacun un atome d'hydrogène ou un groupe alkyle ;
les groupes R^{c} sont identiques ou différents et représentent chacun un groupe alkyle, cycloalkyle, -alkyl-aryle, -alkylcycloalkyle ou aryle facultativement substitué avec R^{a} ;
les groupes R^{d} sont identiques ou différents et représentent chacun un atome d'hydrogène, un groupe alkyle ou aryle facultativement substitué avec R^{a} ;
les groupes R^{e} sont identiques ou différents et représentent chacun un atome d'hydrogène ou un groupe alkyle ; ou bien R^{e} représente un groupe aryle ou hétéroaryle, l'un ou l'autre étant facultativement substitué avec R^{a} ;
un ou deux de T, U, V et W représentent un atome d'azote et les autres représentent chacun un groupe C(R^{a}) ;
les atomes X sont identiques ou différents et représentent chacun un atome d'oxygène ou de soufre ;
Y et Z sont identiques ou différents et représentent chacun un atome d'hydrogène, un atome d'halogène, un groupe alkyle, hydroxy, alkoxy, -CN, -N(R^{d})C(=X)R^{c}, -C(=X)N(R^{c})(R^{d}), -S(O)ₘ-R^{c}, -N(R^{c})(R^{d})S(O)₂, -S(O)₂N(R^{c})(R^{d}), -N(R^{e})₂, -Si(R^{c})₃, -alkyl-Si(R^{c})₃, aryle facultativement substitué avec R^{a} ou -O-aryle facultativement substitué avec R^{a} ;
Y' représente un groupe -Si(R^{c})₃ ou -alkyl-Si(R^{c})₃ ;
les noyaux 1 et 2 sont identiques ou différents et représentent chacun un noyau arylène ou hétéroarylène, l'un ou l'autre étant facultativement substitué avec R^{a} ;
les indices m sont identiques ou différents et sont chacun égaux à 0, 1 ou 2 ; et
les indices n sont identiques ou différents et sont chacun égaux à 0, 1, 2 ou 3 ;
sous réserve que : au moins un de Y et Z comprend un atome de silicium ; le composé ne contient pas une liaison simple N-N ; et le composé n'est pas le 5-[2-bromo-5-(triméthylsilyl)phénoxy]-*N*-{2-[(2-aminoéthyl)propylamino)]-4,6-diméthoxy-1,3-pyrimidine-5-yl}furanne-2-carboxamide ;
ou un de ses sels pharmaceutiquement acceptables.

2. Composé suivant la revendication 1, dans lequel D représente un groupe -O-, -S- ou -CH₂-.

3. Composé suivant la revendication 1 ou la revendication 2, dans lequel E est absent.

4. Composé suivant l'une quelconque des revendications précédentes, dans lequel F représente un groupe -C(O)-.

5. Composé suivant l'une quelconque des revendications précédentes, dans lequel G représente un groupe -N(R^{d})-.

6. Composé suivant la revendication 5, dans lequel R^{d} représente un atome d'hydrogène.

7. Composé suivant l'une quelconque des revendications précédentes, dans lequel J représente un groupe de formule (i) et i, j et k sont respectivement égaux à : 2, 2 et 0 ; 1, 1 et 0 ; 2, 1 et 0 ; ou 2, 1 et 1.

8. Composé suivant la revendication 7, dans lequel i, j et k sont respectivement égaux à 2, 2 et 0 et R^{f} est présent.

9. Composé suivant l'une quelconque des revendications 1 à 6, dans lequel J représente un groupe de formule (ii) et R^{g} représente un groupe éthyle, propyle, éthanoyle ou propanoyle.

10. Composé suivant l'une quelconque des revendications 1 à 6, dans lequel J représente un groupe azabicyclo-[3.2.1]octane-3-yle.

11. Composé suivant l'une quelconque des revendications précédentes, dans lequel K représente un groupe méthyle et L représente un atome d'hydrogène ou un groupe méthyle ; ou bien K-N-L, pris conjointement, forment un groupe azétidinyle.

12. Composé suivant l'une quelconque des revendications précédentes, le composé répondant à n'importe laquelle des formules (I) à (III), dans laquelle un ou deux de T, U, V et W représentent un atome d'azote et les autres représentent chacun un groupe CH.

13. Composé suivant l'une quelconque des revendications précédentes, dans lequel le noyau 1 est un noyau furannylène.

14. Composé suivant l'une quelconque des revendications précédentes, dans lequel le noyau 2 est un noyau phénylène, pyrimidylène ou pyridinylène, n'importe lequel de ces noyaux étant facultativement substitué.

15. Composé suivant la revendication 14, dans lequel le noyau 2 est substitué 1, 2 ou 3 fois, les substituants étant identiques ou différents et étant choisis entre des substituants alkoxy et halogéno.

16. Composé suivant la revendication 1, choisi entre :
N-(2-(4-aminopipéridine-1-yl)-4,6-diméthoxypyrimidine-5-yl)-5-(2-méthyl-5-(triméthylsilyl)phénoxy)furanne-2-carboxamide ;
N-(4,6-diméthoxy-2-(4-(méthylamino)pipéridine-1-yl)-pyrimidine-5-yl)-5-(2-méthyl-5-(triméthylsilyl)phénoxy)furanne-2-carboxamide ;
N-(2-(4-(diméthylamino)pipéridine-1-yl)-4,6-diméthoxy-pyrimidine-5-yl)-5-(2-méthyl-5-(triméthylsilyl)phénoxy)furanne-2-carboxamide ;
N-(2-(4-(azétidine-1-yl)pipéridine-1-yl)-4,6-diméthoxy-pyrimidine-5-yl)-5-(2-méthyl-5-(triméthylsilyl)phénoxy)furanne-2-carboxamide ;
N-(2-(4-(diméthylamino)pipéridine-1-yl)-4,6-diméthoxy-pyrimidine-5-yl)-5-(3-(triméthylsilyl)phénoxy)furanne-2-carboxamide ;
N-(2-(4-(diméthylamino)pipéridine-1-yl)-4,6-diméthoxy-pyrimidine-5-yl)-5-(3-(éthyldiméthylsilyl)phénoxy)furanne-2-carboxamide ;
N-(2-(3-aminoazétidine-1-yl)-4,6-diméthoxypyrimidine-5-yl)-5-(2-méthyl-5-(triméthylsilyl)phénoxy)furanne-2-carboxamide ;
N-(4,6-diméthoxy-2-(3-(méthylamino)azétidine-1-yl)-pyrimidine-5-yl)-5-(2-méthyl-5-(triméthylsilyl)phénoxy)furanne-2-carboxamide ;
N-(2-(3-(diméthylamino)azétidine-1-yl)-4,6-diméthoxy-pyrimidine-5-yl)-5-(2-méthyl-5-(triméthylsilyl)phénoxy)furanne-2-carboxamide ;
N-(2-(3-(azétidine-1-yl)azétidine-1-yl)-4,6-diméthoxy-pyrimidine-5-yl)-5-(2-méthyl-5-(triméthylsilyl)phénoxy)furanne-2-carboxamide ;
N-(2-(3-(azétidine-1-yl)azétidine-1-yl)-4,6-diméthoxy-pyrimidine-5-yl)-5-(3-(triméthylsilyl)phénoxy)furanne-2-carboxamide ;
N-(2-(3-(azétidine-1-yl)azétidine-1-yl)-4,6-diméthoxy-pyrimidine-5-yl)-5-(3-(éthyldiméthylsilyl)phénoxy)furanne-2-carboxamide ;
N-(2-(3-aminopyrrolidine-1-yl)-4,6-diméthoxypyrimidine-5-yl)-5-(2-méthyl-5-(triméthylsilyl)phénoxy)furanne-2-carboxamide ;
N-(4,6-diméthoxy-2-(3-(méthylamino)pyrrolidine-1-yl)-pyrimidine-5-yl)-5-(2-méthyl-5-(triméthylsilyl)phénoxy)furanne-2-carboxamide ;
N-(2-(3-(diméthylamino)pyrrolidine-1-yl)-4,6-diméthoxy-pyrimidine-5-yl)-5-(2-méthyl-5-(triméthylsilyl)phénoxy)furanne-2-carboxamide ;
N-(2-(3-(azétidine-1-yl)pyrrolidine-1-yl)-4,6-diméthoxy-pyrimidine-5-yl)-5-(2-méthyl-5-(triméthylsilyl)phénoxy)furanne-2-carboxamide ;
N-(2-(3-(diméthylamino)pyrrolidine-1-yl)-4,6-diméthoxy-pyrimidine-5-yl)-5-(3-(triméthylsilyl)phénoxy)furanne-2-carboxamide ;
N-(2-(3-(diméthylamino)pyrrolidine-1-yl)-4,6-diméthoxy-pyrimidine-5-yl)-5-(3-(éthyldiméthylsilyl)phénoxy)furanne-2-carboxamide ;
N-(2-(3-(aminométhyl)pyrrolidine-1-yl)-4,6-diméthoxy-pyrimidine-5-yl)-5-(2-méthyl-5-(triméthylsilyl)phénoxy)furanne-2-carboxamide ;
N-(4,6-diméthoxy-2-(3-((méthylamino)méthyl)pyrrolidine-1-yl)pyrimidine-5-yl)-5-(2-méthyl-5-(triméthylsilyl)phénoxy)-furanne-2-carboxamide ;
N-(2-(3-((diméthylamino)méthyl)pyrrolidine-1-yl)-4,6-diméthoxypyrimidine-5-yl)-5-(2-méthyl-5-(triméthylsilyl)-phénoxy)furanne-2-carboxamide ;
N-(2-(3-(azétidine-1-ylméthyl)pyrrolidine-1-yl)-4,6-diméthoxypyrimidine-5-yl)-5-(2-méthyl-5-(triméthylsilyl)-phénoxy)furanne-2-carboxamide ;
N-(2-(3-((diméthylamino)méthyl)pyrrolidine-1-yl)-4,6-diméthoxypyrimidine-5-yl)-5-(3-(triméthylsilyl)phénoxy)-furanne-2-carboxamide ;
N-(2-(3-((diméthylamino)méthyl)pyrrolidine-1-yl)-4,6-diméthoxypyrimidine-5-yl)-5-(3-(éthyldiméthylsilyl)phénoxy)-furanne-2-carboxamide ;
N-(2-(2-(aminométhyl)morpholine-4-yl)-4,6-diméthoxy-pyrimidine-5-yl)-5-(2-méthyl-5-(triméthylsilyl)phénoxy)furanne-2-carboxamide ;
N-(4,6-diméthoxy-2-(2-((méthylamino)méthyl)morpholine-4-yl)pyrimidine-5-yl)-5-(2-méthyl-5-(triméthylsilyl)phénoxy)-furanne-2-carboxamide ;
N-(2-(2-((diméthylamino)méthyl)morpholine-4-yl)-4,6-diméthoxypyrimidine-5-yl)-5-(2-méthyl-5-(triméthylsilyl)phénoxy)-furanne-2-carboxamide ;
N-(2-(2-(azétidine-1-ylméthyl)morpholine-4-yl)-4,6-diméthoxypyrimidine-5-yl)-5-(2-méthyl-5-(triméthylsilyl)phénoxy)-furanne-2-carboxamide ;
N-(2-(2-((diméthylamino)méthyl)morpholine-4-yl)-4,6-diméthoxypyrimidine-5-yl)-5-(3-(triméthylsilyl)phénoxy)furanne-2-carboxamide ;
N-(2-(2-((diméthylamino)méthyl)morpholine-4-yl)-4,6-diméthoxypyrimidine-5-yl)-5-(3-(éthyldiméthylsilyl)phénoxy)-furanne-2-carboxamide ;
N-(2-(4-(diméthylamino)-2-oxopipéridine-1-yl)-4,6-diméthoxy-pyrimidine-5-yl)-5-(2-méthyl-5-(triméthylsilyl)phénoxy)furanne-2-carboxamide ;
N-(4,6-dyméthoxy-2-(4-(méthylamino)-2-oxopipéridine-1-yl)pyrimidine-5-yl)-5-(2-méthyl-5-(triméthylsilyl)phénoxy)-furanne-2-carboxamide ;
N-(2-(4-(diméthylamino)-2-oxopipéridine-1-yl)-4,6-diméthoxy-pyrimidine-5-yl)-5-(3-(triméthylsilyl)phénoxy)furanne-2-carboxamide ;
N-(4,6-diméthoxy-2-(4-(méthylamino)-2-oxopipéridine-1-yl)pyrimidine-5-yl)-5-(3-(triméthylsilyl)phénoxy)furanne-2-carboxamide ;
N-(2-(4-(diméthylamino)-2-oxopipéridine-1-yl)-4,6-diméthoxy-pyrimidine-5-yl)-5-(3-(éthyldiméthylsilyl)phénoxy)furanne-2-carboxamide ;
N-(2-((2-aminoéthyl)(propyl)amino)-4,6-diméthoxypyrimidine-5-yl)-5-(2-méthyl-5-(triméthylsilyl)phénoxy)furanne-2-carboxamide ;
N-(4,6-diméthoxy-2-((2-(méthylamino)éthyl)(propyl)amino)-pyrimidine-5-yl)-5-(2-méthyl-5-(triméthylsilyl)phénoxy)furanne-2-carboxamide ;
N-(2-((2-(diméthylamino)éthyl)(propyl)amino)-4,6-diméthoxy-pyrimidine-5-yl)-5-(2-méthyl-5-(triméthylsilyl)phénoxy)furanne-2-carboxamide ;
N-(2-((2-(azétidine-1-yl)éthyl)(propyl)amino)-4,6-diméthoxy-pyrimidine-5-yl)-5-(2-méthyl-5-(triméthylsilyl)phénoxy)furanne-2-carboxamide ;
N-(2-((2-(diméthylamino)éthyl)(propyl)amino)-4,6-diméthoxy-pyrimidine-5-yl)-5-(3-(triméthylsilyl)phénoxy)furanne-2-carboxamide ;
N-(2-((2-(diméthylamino)éthyl)(propyl)amino)-4,6-diméthoxy-pyrimidine-5-yl)-5-(3-(éthyldiméthylsilyl)phénoxy)furanne-2-carboxamide ;
N-(2-((2-aminoéthyl)(éthyl)amino)-4,6-diméthoxypyrimidine-5-yl)-5-(2-méthyl-5-(triméthylsilyl)phénoxy)furanne-2-carboxamide ;
N-(2-(éthyl(2-(méthylamino)éthyl)amino)-4,6-diméthoxy-pyrimidine-5-yl)-5-(2-méthyl-5-(triméthylsilyl)phénoxy)furanne-2-carboxamide ;
N-(2-((2-(diméthylamino)éthyl)(éthyl)amino)-4,6-diméthoxy-pyrimidine-5-yl)-5-(2-méthyl-5-(triméthylsilyl)phénoxy)furanne-2-carboxamide ;
N-(2-((2-(azétidine-1-yl)éthyl)(éthyl)amino)-4,6-diméthoxy-pyrimidine-5-yl)-5-(2-méthyl-5-(triméthylsilyl)phénoxy)furanne-2-carboxamide ;
N-(2-((2-(diméthylamino)éthyl)(éthyl)amino)-4,6-diméthoxy-pyrimidine-5-yl)-5-(3-(triméthylsilyl)phénoxy)furanne-2-carboxamide ;
N-(2-((2-(diméthylamino)éthyl)(éthyl)amino)-4,6-diméthoxy-pyrimidine-5-yl)-5-(3-(éthyldiméthylsilyl)phénoxy)furanne-2-carboxamide ;
N-(2-(N-(2-(diméthylamino)éthyl)propionamido)-4,6-diméthoxy-pyrimidine-5-yl)-5-(2-méthyl-5-(triméthylsilyl)phénoxy)furanne-2-carboxamide ;
N-(2-(N-(2-(diméthylamino)éthyl)acétamido)-4,6-diméthoxy-pyrimidine-5-yl)-5-(2-méthyl-5-(triméthylsilyl)phénoxy)furanne-2-carboxamide ;
N-(2-(N-(2-(diméthylamino)éthyl)propionamido)-4,6-diméthoxy-pyrimidine-5-yl)-5-(3-(triméthylsilyl)phénoxy)furanne-2-carboxamide ;
N-(2-(N-(2-(diméthylamino)éthyl)acétamido)-4,6-diméthoxy-pyrimidine-5-yl)-5-(3-(triméthylsilyl)phénoxy)furanne-2-carboxamide ;
N-(2-(N-(2-aminoéthyl)proprionamido)-4,6-diméthoxypyrimidine-5-yl)-5-(2-méthyl-5-(triméthylsilyl)phénoxy)furanne-2-carboxamide ;
N-(2-(N-(2-aminoéthyl)acétamido)-4,6-diméthoxypyrimidine-5-yl)-5-(2-méthyl-5-(triméthylsilyl)phénoxy)furanne-2-carboxamide ;
5-(2-méthyl-5-(triméthylsilyl)phénoxy)-N-(2-(3-(diméthyl-amino)-8-azabicyclo[3.2.1]octane-8-yl)-4,6-diméthoxypyrimidine-5-yl)furanne-2-carboxamide ;
N-(2-(éthyl(2-(isopropylamino)éthyl)amino)-4,6-diméthoxy-pyrimidine-5-yl)-5-(2-méthyl-5-(triméthylsilyl)phénoxy)furanne-2-carboxamide ;
N-(2-(3-(isopropylamino)pyrrolidine-1-yl)-4,6-diméthoxy-pyrimidine-5-yl)-5-(2-méthyl-5-(triméthylsilyl)phénoxy)furanne-2-carboxamide ;
*N*-(4,6-diméthoxy-2-(méthyl(1-méthylpyrrolidine-3-yl)-amino)pyrimidine-5-yl)-5-(2-méthyl-5-(triméthylsilyl)phénoxy)-furanne-2-carboxamide ; et
N-(4,6-diméthoxy-2-(imidazolidine-2-one-1-yl)pyrimidine-5-yl)-5-(2-méthyl-5-(triméthylsilyl)phénoxy)furanne-2-carboxamide.

17. Composé suivant l'une quelconque des revendications précédentes, qui est sous forme d'un énantiomère, diastéréoisomère
ou tautomère unique.

18. Composé suivant l'une quelconque des revendications précédentes, à usage thérapeutique.

19. Composition pharmaceutique comprenant un composé de l'une quelconque des revendications 1 à 18 et un diluant
ou support pharmaceutiquement acceptable.

20. Utilisation d'un composé suivant l'une quelconque des revendications 1 à 17 pour la production d'un médicament destiné à la thérapie du cancer.

21. Utilisation d'un composé suivant l'une quelconque des revendications 1 à 17 pour la production d'un médicament destiné au traitement ou à la prévention de l'endométriose, du myome utérin, d'une maladie ovarienne, d'une maladie kystique mammaire, de l'hypertrophie prostatique, de l'aménorrhée, de la puberté précoce, du syndrome prémenstruel, d'une physiopathologie dépendant de stéroïdes sexuels ou de l'hyperplasie prostatique bénigne, ou pour l'interruption de la spermatogenèse.

22. Utilisation suivant la revendication 21, pour le traitement ou la prévention de l'endométriose avec une douleur, de la maladie ovarienne polykystique ou de l'aménorrhée secondaire.

23. Utilisation d'un composé suivant l'une quelconque des revendications 1 à 17 pour la production d'un médicament destiné au traitement ou à la prévention de la maladie d'Alzheimer.

24. Utilisation d'un composé suivant l'une quelconque des revendications 1 à 17 pour la production d'un médicament destiné au traitement ou à la prévention de l'infection par le VIH ou du SIDA.

25. Utilisation d'un composé suivant l'une quelconque des revendications 1 à 17 pour la production d'un médicament destiné au traitement ou à la prévention d'une maladie provoquée par un dysfonctionnement thymique.

26. Utilisation suivant la revendication 25, pour le traitement ou la prévention de la sclérose en plaques, de la polyarthrite rhumatoïde ou du diabète de type 1.
